Europäisches Patentamt

European Patent Office

Office européen des brevets

(11) Publication number: **0 366 239**
**A1**

## EUROPEAN PATENT APPLICATION

(21) Application number: 89308787.4

(51) Int. Cl.⁵: **C12P 21/02 , A61K 39/145 ,**
**//C12N15/44**

(22) Date of filing: 30.08.89

The title of the invention has been amended (Guidelines for Examination in the EPO, A-III, 7.3).

(30) Priority: 31.08.88 US 238803
28.07.89 US 387558

(43) Date of publication of application:
02.05.90 Bulletin 90/18

(84) Designated Contracting States:
AT BE CH DE ES FR GB GR IT LI LU NL SE

(71) Applicant: SMITHKLINE BEECHAM CORPORATION
One Franklin Plaza P.O. Box 7929
Philadelphia Pennsylvania 19103(US)

(72) Inventor: Young, James Francis
12624 Gravenhurst Lane
Gaithersburg MD 20878(US)
Inventor: Jones, Christopher S.
709 Roberts Drive
Stowe Pennsylvania 19464(US)

(74) Representative: Giddings, Peter John, Dr. et al
Smith Kline & French Laboratories Ltd.
Corporate Patents Mundells
Welwyn Garden City Hertfordshire AL7 1EY(GB)

(54) Purification process for recombinant influenza proteins.

(57) A process for purifying an immunogenic polypeptide for protection against influenza virus infection, said polypeptide comprising an immunogenic determinant of the HA2 subunit of an HA protein.

EP 0 366 239 A1

## PURIFICATION PROCESS

### FIELD OF THE INVENTION

This invention relates to vaccine preparation and, more particularly, to preparation of a vaccinal influenza virus polypeptide by recombinant DNA techniques.

### BACKGROUND OF THE INVENTION

Influenza virus infection causes acute respiratory disease in man, horses and fowl, sometimes of pandemic proportions. Influenza viruses are orthomyxoviruses and, as such, have enveloped virions of 80 to 120 nanometers in diameter, with two different glycoprotein spikes. Three types, A, B and C, infect humans. Type A viruses have been responsible for the majority of human epidemics in modern history, although there are also sporadic outbreaks of Type B infections. Known swine, equine and fowl viruses have mostly been type A, although type C viruses have also been isolated from swine.

The type A viruses are divided into subtypes based on the antigenic properties of the hemagglutinin (HA) and neuraminidase (NA) surface glycoproteins. Within type A, subtypes H1 ("swine flu"), H2 ("asian flu") and H3 ("Hong Kong flu") are predominant in human infections.

Due to genetic drift which, at approximately yearly intervals, affects antigenic determinants in the HA and NA proteins, it has not been possible to prepare a "universal" influenza virus vaccine using conventional killed or attenuated viruses, that is, a vaccine which is non strain specific. Recently, attempts have been made to prepare such universal, or semi-universal, vaccines from reassortant viruses prepared by crossing different strains. More recently, such attempts have involved recombinant DNA techniques focusing primarily on the HA protein.

### REPORTED DEVELOPMENTS

Winter et al., Nature, volume 292, pages 72-75 (1981), report a DNA coding sequence for HA of the A/PR/8/34 strain (H1N1) consisting of a 17 residue hydrophobic signal peptide, an HA1 subunit (326 residues long) and an HA2 subunit (222 residues long) separated by a single arginine residue (327) thought to be recognized during processing by a trypsin-like enzyme. Percent homology of amino acid and nucleotide sequences of the HA1 and HA2 subunits of this strain were compared to those of representative strains of subtypes H2, H3 and H7.

Baez et al., Nucl. Acids Res., volume 8, pages 5845-5857 (1980), report a DNA coding sequence for the nonstructural (NS) protein of strain A/PR/8/34.

Young et al., in The Origin of Pandemic Influenza Viruses, 1983, edit. by W.G. Laver, Elsevier Science Publishing Co., and Young et al., Proc. Natl. Acad. Sci. USA, volume 80, pages 6105-6109 (1983), report cloning of cDNA from all eight RNA segments from strain A/PR/8/34 in E. coli and report high level expression of the NS1 protein in E. coli.

Emtage et al., U.S. Patent 4,357,421, disclose cloning and expression of a coding sequence for an influenza virus HA gene, and disclose that the HA polypeptide is an antigen which may be administered for vaccine purposes.

The Morbidity and Mortality Weekly Report, volume 33, number 19, pages 253-261, reviews the most recent prevention and control strategies for influenza virus, including dosage and administration protocols for HA protein-containing human vaccines.

Davis et al., Gene, volume 21, pages 273-284 (1983) report on immune responses in mice to HA-derived polypeptides.

Additional references report cloning and expression of HA, NS and other influenza virus genes of the A/PR/8/34 and other strains. Some of such references are cited herein below.

### DESCRIPTION OF THE DRAWINGS

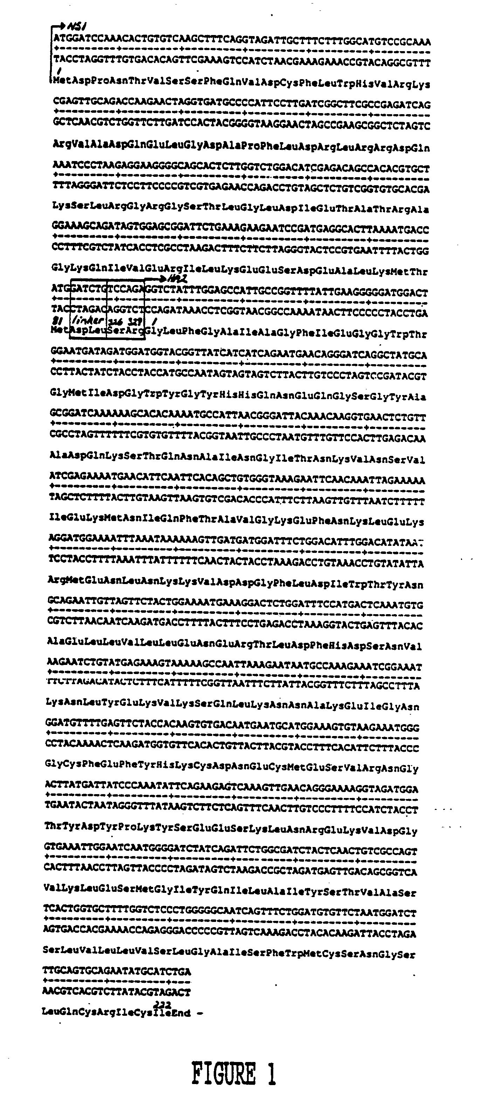

FIGURE 1 is the nucleotide sequence of the coding region for C13 protein and the amino acid sequence thereof. The boxed region indicates the sequence joining the C-terminal amino acid 81 (methionine) of NS1 and the N-terminal amino acid 1 (glycine) of the entire HA2 subunit (amino acids 1-222).

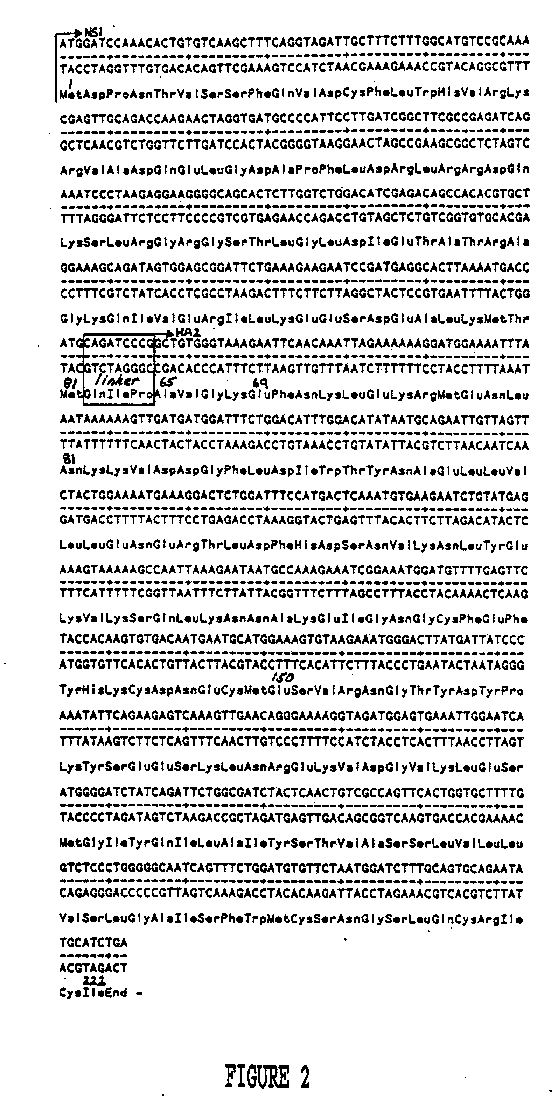

FIGURE 2 is the nucleotide sequence of the codomg region for D protein and the amino acid sequence thereof. The boxed region indicates the linker sequence between the C-terminal amino acid 81 (methionine) of NS1 and the N-terminal amino acid 65 (alanine) of the truncated HA2 subunit. The positions of amino acids 69 (glutamic acid), 81 (asparagine) and 150 (glutamic acid) of HA2, corresponding to the N-terminal amino acids of the truncated HA2 subunit in "A" protein, "C" protein and "ΔD" protein, respectively, are also indicated.

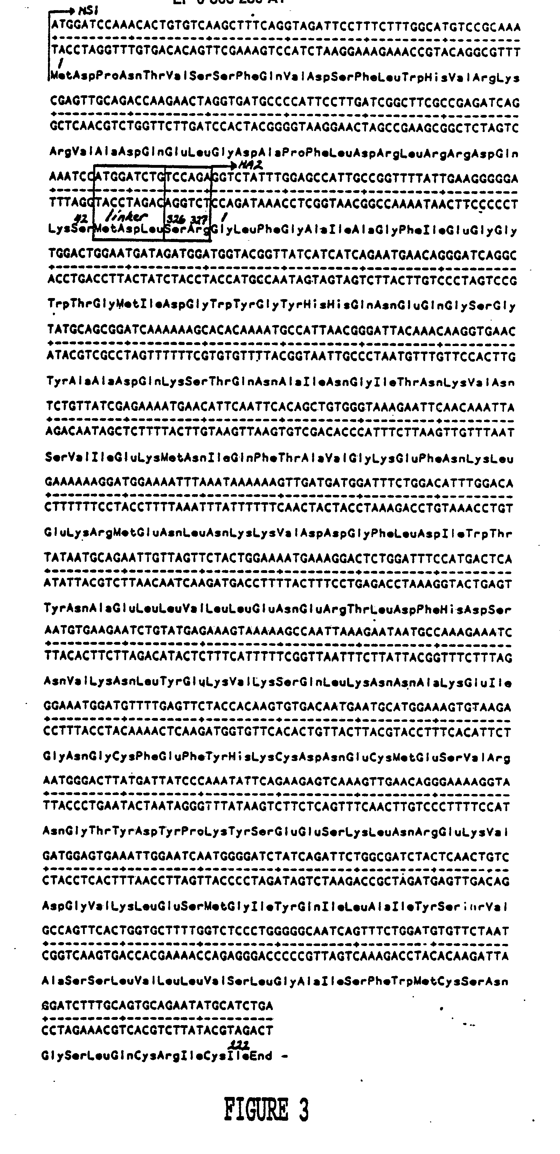

FIGURE 3 is the nucleotide sequence of the coding region for C13 short protein and the amino acid sequence thereof. The boxed region indicates the sequence joining the C-terminal amino acid 42 (serine) of NS1 and the N-terminal amino acid 1 (glycine) of the entire HA2 subunit. Amino acid 13 of NS1 (cysteine) has been replaced by serine.

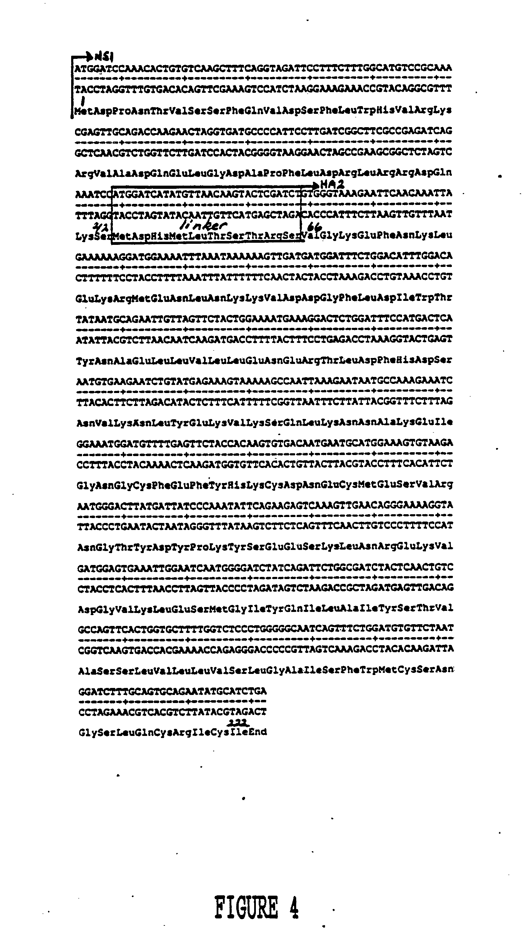

FIGURE 4 is the nucleotide sequence of the coding region for D short protein and the amino acid sequence thereof. The boxed region indicates the linker sequence between the C-terminal amino acid 42 (serine) of NS1 and the N-terminal amino acid 66 (valine) of the truncated HA2 subunit. Amino acid 13 of NS1 (cysteine) has been replaced by serine.

## SUMMARY OF THE INVENTION

In one aspect, the invention is a vaccine for stimulating protection in animals against infection by influenza virus which comprises a polypeptide, other than an HA protein, having an immunogenic determinant of the HA2 subunit of an HA protein.

In another aspect, the invention is a polypeptide, other than an HA protein, which comprises an immunogenic determinant of the HA2 subunit, which can be used in the vaccine of the invention.

In still another aspect, the invention relates to a process for purifying the polypeptides of the invention from the cell lysate of a recombinant host cell culture, the process comprising:

subjecting the cell lysate to a first detergent treatment, at a pH in the range between about 6 to about 8.5, to selectively solubilize host cell contaminants and forming thereby a soluble fraction and an insoluble fraction, the insoluble fraction containing the polypeptide;

separating the soluble fraction from the insoluble fraction;

subjecting the insoluble fraction to a second detergent treatment, at a pH in the range between about 9 to about 11, to selectively solubilize host cell contaminants and forming thereby a soluble fraction and an insoluble fraction, the insoluble fraction containing the polypeptide;

separating the soluble fraction from the insoluble fraction;

subjecting the insoluble fraction to a chaotropic agent thereby solubilizing the polypeptide;

separating the soluble fraction from the insoluble fraction, the soluble fraction containing the polypeptide;

adding a reducing agent to the soluble fraction; and

subjecting the soluble fraction to ion exchange chromatography and recovering the polypeptide -containing eluate, said eluate being substantially free of contaminating host cell nucleic acids, endotoxin and polypeptides.

In other aspects, the invention is a DNA molecule comprising a coding sequence for the vaccinal polypeptide of the invention, including the coding sequence alone or incorporated into a larger molecule, such as a DNA cloning or expression vector, and a microorganism or cell transformed with such DNA molecule.

## DETAILED DESCRIPTION OF THE INVENTION

Oftentimes, immunogenic determinants do not stimulate an immunoprotective response. This is believed to be due, in large part, to a failure to present the determinant to a host's bodily defense system in proper configuration.

As disclosed and fully described herein below, the immunogenic determinant (which may comprise one or more contiguous or separated haptens) of the HA2 subunit of the HA protein, surprisingly, induces a cytotoxic T cell response against different strains within the subtype of origin. Thus, the HA2 immunogenic determinant can provoke a protective immune response, if it is presented in an immunogenic configuration, which is subtype specific, rather than strain specific. For example, the HA2 determinant can be presented in a vaccinal polypeptide comprising the HA2 subunit, that is, substantially the entire HA2 subunit of the HA protein, fused to a second polypeptides which permits an immune response to the immunogenic determinant by causing the HA2 subunit to assume an immunogenic configuration.

Preferably, the polypeptide which permits such immune response to the HA2 immunogenic determinant comprises an amino acid sequence which is expressed at high levels by a recombinant host, prokaryotic or eukaryotic, fused to the N terminus of substantially the entire HA2 subunit. Especially preferred is a polypeptide derived from an influenza virus protein. The vaccinal polypeptide is not the HA protein, because immunoprotective response to the HA protein appears to be strain-specific.

For expression of such vaccinal polypeptide carrying the HA2 immunogenic determinant in E. coli, the polypeptide which permits an immune response to the HA2 determinant is preferably the N terminus of the NS1 influenza virus protein. A particular and preferred embodiment thereof is a protein herein referred to as C13. As depicted in Figure 1, C13 protein has the first 81 amino acids of the NS1 protein fused, via a linker sequence which codes for aspartate-leucine, to a peptide comprising amino acid 326 (serine) of the HA1 subunit of the HA protein, amino acid 327 (arginine) of HA separating HA1 from HA2, and the entire HA2 subunit (amino acids 1-222). (Numbering of HA amino acids from Winter et al., Nature, 292:72 (1981)).

In another embodiment, the vaccinal polypeptide is a protein referred to as "C13 short". As depicted in Figure 3, C13 short comprises the first 42 amino acids of the NS1 protein (with the exception that amino acid 13 (cysteine) is replaced by serine) fused, via a linker sequence which codes for methionine-aspartate-leucine, to a peptide comprising amino acid 326 (serine) of the HA1 subunit of the HA protein, amino acid 327 (arginine) of HA separating HA1 from HA2, and the entire HA2 subunit (amino acids 1-222).

More preferred because of its relative ease of isolation and purification is D protein. As depicted in Figure 2, D protein comprises the first 81 amino acids of NS1 fused, via a linker sequence which codes for glutamine-isoleucine-proline, to N-terminal amino acid 65 of the truncated HA2 subunit (amino acids 65 222). A DNA coding sequence for D protein is prepared by restricting the HA2 coding sequence with Pvu II and ligating the C-terminal region of the NcoI site between amino acids 81 and 82 in the NS1 coding sequence via a synthetic oligonucleotide linker.

In other embodiments, the vaccinal polypeptides comprise derivatives of D protein, namely, "A" protein and "C" protein wherein the first 81 amino acids of NS1 are fused, via a linker sequence coding for glutamine-isoleucine-proline, to N terminal amino acid 69 of the truncated HA2 subunit (amino acids 69 222) (A protein), and N-terminal amino acid 81 of the truncated HA2 subunit (amino acids 81-222) (C protein), respectively. A third D protein derivative is ΔD protein which comprises the first 81 amino acids of NS1 fused, via a linker sequence coding for glutamine-isoleucine-proline-valine to N-terminal amino acid 150 of the truncated HA2 subunit (amino acids 150-222).

In yet another embodiment, the vaccinal polypeptide is a protein referred to as "D short". As depicted in Figure 4, D short comprises the first 42 amino acids of the NS1 protein (with the exception that amino acid 13 (cysteine) is replaced by serine) fused, via a linker sequence which codes for methionine-asparatate-histidine-methionine-leucine-threonine-serine-threonine-arginine-serine, to N-terminal amino acid 66 of the truncated HA2 subunit (amino acids 66-222).

The vaccinal polypeptides of the invention can be prepared by chemical synthesis techniques. Preferably, however, they are prepared by known recombinant DNA techniques by cloning and expressing within a host microorganism or cell a DNA fragment carrying a coding sequence for the polypeptide. The preferred host is E. coli because it can be used to produce large amounts of desired proteins safely and cheaply.

Coding sequences for the HA2, NS1 and other viral proteins of influenza virus can be prepared synthetically or can be derived from viral RNA by known techniques, or from available cDNA-containing plasmids. For example, in addition to the above-cited references, a DNA coding sequence for HA from the A/Japan/305/57 strain was cloned, sequenced and reported by Gething et al., Nature, volume 287, pages 301-306 (1980); an HA coding sequence for strain A/NT/60/68 was cloned as reported by Sleigh, et al., and by Both et al., both in Developments in Cell Biology, Elsevier Science Publishing Co., pages 69-79 and 81-89, 1980; an HA coding sequence for strain A/WSN/33 was cloned as reported by Davis et al., Gene, volume 10, pages 205-218 (1980); and by Hiti et al., Virology, volume 111, pages 113-124 (1981). An HA coding sequence for fowl plague virus was cloned as reported by Porter et al. and by Emtage et al., both in Developments in Cell Biology, cited above, at pages 39-49 and 157-168. Also, influenza viruses including other strains, subtypes and types, are available from clinical specimens and from public depositories, such as the American Type Culture Collection, Rockville, Maryland, U.S.A.

Systems for cloning and expression the vaccinal polypeptide in various microorganisms and cells, including, for example, E. coli, Bacillus, Streptomyces, Saccharomyces, mammalian and insect cells, are known and available from private and public laboratories and depositories and from commercial vendors.

The process of the present invention involves subjecting a cell lysate, derived from a recombinant host cell culture and containing pyrogenic, proteinaceous and nucleic acid contaminants of cellular origin, to a series of solubilization and separation procedures, including centrifugation and chromatography, to yield the desired polypeptides substantially free of contaminants.

The recombinant host is prepared as described hereinbelow. Such recombinant cells are cultured in nutrient media containing assimilable sources of carbon, nitrogen and minerals, in the presence of oxygen, by standard fermentation techniques. Following fermentation for a time sufficient to express the recombinant polypeptide, cells are collected, such as by centrifugation or filtration. The resulting cell paste is then resuspended and subjected to lysis.

Cell lysis can be accomplished by addition of lysozyme or other lysing or permeabilizing agent to a buffered suspension (between pH 6 and pH 8.5, pH 8 being preferred) of the cell pellet at a cell concentration of about 100-300 g/L, based on the wet cell pellet weight.

The weight of the cell pellet in production scale operation may rather from 800-3,000 g, depending on the particular polypeptide undergoing purification. A suitable lysis buffer is Tris (50mM), EDTA (2mM), dithiothreitol (DTT) (0.1 mM), and glycerol (5%) having a pH of about 8.0. Lysis may also be performed by mechanical or ultrasonic disruption means in the absence of lysozyme. Satisfactory results have been obtained using a Gaulin homogenizer (APV Gaulin, Inc., Everett, Massachusetts). A combination of chemical, mechanical and/or ultrasonic lysing means may be employed, if desired.

The lysed suspension is then treated, at a pH in the range of between about 6 and 8.5, pH 8 being preferred, with a detergent, for example, an ionic detergent such as deoxycholate, e.g. sodium salt monohydrate (approx. 0.1%), to prevent binding of the desired recombinant polypeptide to the cell debris (membranes and proteins) and/or a non-ionic detergent, which dissolves cytoplasmic membranes without denaturing protein, such as Triton X-100 (International Biotechnologies, Inc., New Haven, Connecticut). The lysed suspension is clarified, e.g., by continuous centrifugation at about 25,000 Xg in a Beckman JCF-G rotor at a flow rate of 100-500 ml/min, the supernatant containing solubilized host cell contaminants discarded and the insoluble fraction containing the polypeptide subjected to further purification.

The cell lysate may be subjected to additional detergent treatments for the removal of still other host cell contaminants. For example, the pellet containing the polypeptide may be resuspended in a suitable buffer at a pH in the range of between about 9.5 to about 11. A particularly useful buffer is glycine-NaOH (50 mM), EDTA (2mM) and glycerol (5%), at a pH of between 9.5 to 11, preferably 10 to 10.5 pH, 10.5 being most preferred. Resuspension may be facilitated using a homogenizer. A non-ionic detergent such as Triton X -100 may be added to the suspension in order to solubilize additional host cell membranes complexed with the polypeptide which were not earlier solubilized at the lower pH of the previous detergent-treatment step. The suspension is clarified by centrifugation (25,000 Xg) and the supernatant containing solubilized host cell contaminants discarded and the pelleted insoluble fraction containing the polypeptide subjected to further treatment.

Following treatment with detergents, the pellet containing the recombinant polypeptide is treated with a suitable chaotropic agent, for example, urea or guanidine hydrochloride, urea being preferred. Where urea is used as the chaotropic agent, the pellet containing the recombinant polypeptide is dissolved in a urea buffer, for example, 8M urea in 50 mM Tris at a pH between 7.5 to 9, preferably pH 8. Insoluble host cell contaminants are removed by centrifugation, for example, 25,000 xg. The solubilized polypeptide remains in the supernatant.

Partial purification of the cell lysate in the manner just described, significantly reduces the amount of host cell contaminants initially present in the cell lysate.

Further separation of residual host cell contaminants, particularly nucleic acids and endotoxin, from the partially purified polypeptide may be carried out by ion exchange chromatography using a microparticulate column packing having anion exchange groups, such as diethylaminoethyl (DEAE), quarternary aminoethyl (QAE), and polyethylene imine (PEI) bound to a suitable matrix. The ion exchanger should provide a sufficiently porous, open matrix for passage of the polypeptide to be purified. A significant reduction of nucleic acids, endotoxins and, to a lesser extent, low molecular weight host cell contaminating proteins, from a partially purified cell lysate containing the polypeptide has been accomplished on an anion exchange support, for example, a DEAE Fast Flow Sepharose column, equilibrated with the same buffer in which the recombinant protein is solubilized, for example, 8M urea, 50 mM Tris at pH 8.

The addition of a reducing agent, for example, dithiothreitol or 2-mercaptoethanol, to the buffer solution, aids in the removal by ion exchange chromatography of host cell proteins and low molecular weight cleavage products.

The solution containing the peptide is contacted with the ion exchange support and then eluted therefrom. Elution can be carried out using a suitable buffer solution which provides a fraction containing the desired immunogenic polypeptide, substantially free of nucleic acids and contaminating host cell proteins. The buffer solutions used as eluents herein are those widely used in ion exchange chromatography of biological substances. Gradient elution from a anion exchange support is used to advantage with the polypeptides of the present invention, for example, a 0.0 to 0.5 M NaCl gradient, preferably, 0.0 to 0.3 M NaCl, over a 5-fold column volume or greater. The host cell contaminants and the desired polypeptide are adsorbed on the ion exchange matrix, and the desired polypeptide is differentially desorbed in fractions separate from the contaminants. The protein-containing eluate may be passed several times in succession through the same ion exchange column, or through separate columns having different packings.

A further significant reduction of nucleic acids, low molecular weight host cell contaminating proteins and, particularly, endotoxins from the purified cell lysate recovered from anion exchange chromatography (for example, DEAE Fast Flow Sepharose) described immediately above, may be effected by treating the lysate with a strong denaturant such as sodium dodecyl sulfate (SDS) in a reducing environment (achieved by the addition of a reducing agent, for example, dithiothreitol). It is theorized that the denaturant causes the protein of interest to unfold and the reducing environment breaks the protein's disulfide bonds to permit even greater unfolding, thereby solubilizing host cell contaminants believed to be aggregated with the protein. Chromatography of the thus-treated cell lysate by size exclusion chromatography, for example, Bio-Gel A and Bio-Gel PC (Bio-Rad, Richmond, CA USA); Superose 12, Sephadex, Sephacryl-HR, Sepharose and Superdex (Pharmacia), Superose 12 (Pharmacia) chromatography being particularly preferred, results in the elution of a protein-containing fraction of substantially greater purity by removing the host cell contaminants previously aggregated therewith. Still further reductions of host cell contaminants may be effected by repeating treatment of the purified cell lysate with strong denaturants and reducing agents followed by size exclusion chromatography.

To effect removal of the denaturant described above from the purified cell lysate, the protein-containing fractions recovered from the size exclusion chromatography may be subjected to additional size exclusion chromatography. It has been discovered that chromatography columns known in the art as "desalting columns" for example, G50 or preferably G25 Sephadex (Pharmacia) fine chromatography columns may be used to advantage to remove denaturants, particularly where the denaturant is SDS. Denaturant removal is particularly enhanced when done in the presence of a chaotropic agent, for example, urea. By way of example, application of SDS-containing fractions to a desalting column previously equilibrated with a buffer solution containing 8 M urea and subsequent elution of such fractions with the equilibration buffer has been found particularly effective in maximizing recovery of the protein free from contamination by SDS.

The cell lysate, after treatment for removal of contaminating polypeptides, endotoxin and nucleic acids, as described above, is rendered substantially free of residual host cell contaminants.

Various other procedures can be employed in connection with the process of the present invention, although such other procedures are not necessary to achieve a highly purified, pharmaceutical grade product. Such procedures can be employed between, before or after the above described process steps. One such optimal step is diafiltration.

The term "diafiltration" is used herein in the art-recognized sense, to refer to a form of continuous dialysis which is extremely effective in achieving many buffer exchanges. Diafiltration is preferably carried out across a cellulosic membrane or ultrafilter. Suitable membranes/filters are those having from about a 1000 molecular weight (MW) cut-off to those having pore size up to 2.4 um diameter. A number of different systems adaptable to diafiltration are commercially available, such as the 10K Amicon dual spiral cartridge system. In the process of the present invention, diafiltration using a 20 mM Tris buffer at about pH 8 can be effectively employed in the purification and subsequent concentration of the polypeptide.

The vaccine of the invention comprises one or more vaccinal polypeptides of the invention, and a carrier or diluent therefore. For example, such vaccine can comprise substantially the entire HA2 subunit from each of several subtypes, each fused to N terminal amino acids of the NS1 protein, which is highly conserved, in normal saline or other physiological solution. Use of an adjuvant, such as aluminum hydroxide, may prove to be desirable. A preferred vaccine comprises three vaccinal polypeptides, each comprising substantially the entire HA2 subunit from one of the H1, H2 and H3 subtypes fused to about the first 81 amino acids of any NS1 protein, as in the case of the C13 protein. Alternatively, a polyvalent vaccine can be prepared from one or more polypeptides of the invention combined with additional immunogens derived from influenza virus or other pathogens, such as subunit or polypeptide antigens or killed viruses or bacteria, to produce a vaccine which can stimulate protection against influenza virus as well as other invasive organisms or viruses. Techniques for formulating such vaccines are well known. For example, the vaccinal polypeptide, and other immunogens in the case of a combination vaccine, can be lyophilized for subsequent rehydration in saline or other physiological solutions.

Dosage and administration protocol can be optimized in accordance with standard vaccination practices. Typically, the vaccine will be administered intramuscularly, although other routes of administration may be used, such as oral, intraocular, intradermal and intranasal administration. Based on what is known about other polypeptide vaccines, it is expected that a useful single dosage for average adult humans is in the range of 1 to 1000 micrograms, preferably 5 to 150 micrograms, most preferably 10 to 100 micrograms. The vaccine can be administered initially in late summer or early fall and can be readministered two to six weeks later, if desirable, or periodically as immunity wanes, for example, every two to five years.

The following examples are illustrative, and not limiting, of the invention.


Example 1. Plasmid pM30


Plasmid pAPR701 is a pBR322-derived cloning vector which carries coding regions for the M1 and M2 influenza virus proteins (A/PR/8/34). It is described by Young et al., in The Origin of Pandemic Influenza Viruses, 1983, edited by W.G. Laver, Elsevier Science Publishing Co.

Plasmid pAPR801 is a pBR322 derived cloning vector which carries the NS1 coding region (A/PR/8/34). It is described by Young et al., cited above.

Plasmid PAS1 is a pBR322-derived expression vector which contains the $P_L$ promoter, an N utilization site (to relieve transcriptional polarity effects in the presence of N protein) and the cII ribosome binding site including the cII translation initiation codon followed immediately by a BamHI site. It is described by Rosenberg et al., Methods Enzymol., volume 101, pages 123-138 (1983).

Plasmid pAS1deltaEH was prepared by deletion a non-essential EcoRI-HindIII region of pBR322 origin from pAS1. A 1236 base pair Bam HI fragment of pAPR801, containing the NS1 coding region in 861 base pairs of viral origin and 375 base pairs of pBR322 origin, was inserted into the BamHI site of pAS1deltaEH. The resulting plasmid, pAS1deltaEH/801 expresses authentic NS1 (230 amino acids). This plasmid has an Nco I site between the codons for amino acids 81 and 82 and an Nru I site 3' to the NS sequences. The Bam HI site between amino acids 1 and 2 is retained.

A 571 base pair fragment carrying a coding for the C terminal 50 amino acids of the M1 protein was obtained by restricting pAPR 701 with NcoI and EcoRV. This fragment was inserted between the NcoI and NruI sites in pAS1deltaEH/801 subsequent to deletion of that fragment from the plasmid. The resulting plasmid, pM 30, codes for a fusion protein which is the first 81 amino acids of NS1 fused to the last 50 amino acids of M1. The NcoI and BamHI sites are retained.


Example 2. Plasmid pC13


Plasmid pJZ102 is a pBR322-derived cloning vector which carries a coding region for the entire HA protein (A/PR/8/34). It is described by Young et al., cited in Example 1.

Plasmid pBgIII is a pBR322-derived cloning vector which carries a BgIII linker at the NruI site in pBR322.

pJZ102 was cut with Mn1I. BgIII linkers were ligated to all ends and the HA2-containing fragment was inserted into pBgIII. The 5' junction in the resulting plasmid, pBgIII/HA2, was sequenced as follows:

$$\underline{\phantom{xx}\textbf{1}\phantom{xx}} \quad \underline{\phantom{xx}\textbf{2}\phantom{xx}} \quad \underline{\phantom{xx}\textbf{3}\phantom{xx}}$$

$$\textbf{5' \quad AGATCTG \quad TCCAGA \quad GGT\_\_\_ \quad 3'}$$

Region 1 is derived from the Bgl II linker and codes for aspartate and leucine. Region 2 is derived from HA1 and codes for serine (HA1 amino acid 326) and arginine (HA amino acid 327 separating HA1 from HA2). Region 3 is derived from HA2 and codes for all amino acids of the HA2 subunit.

The 3' junction was sequenced as follows:

```
        3         4       5          6
5'  _ _ _ATATGCATC  TGA  GATTAGAATTTCA  CAGATCT
```

Region 4 is the HA2 stop codon. Region 5 is 3' non-coding sequences of viral origin. Region 6 is derived from the Bgl II linker.

A 691 base pair fragment carrying the HA2 coding sequence was obtained by restricting pBgl II/HA2 with BglII. The fragment was end-filled with DNA polymerase I (Klenow) and ligated into pAS1deltaEH/801 which had been cut with Nco I and similarly end-filled (Klenow). The resulting plasmid is pC13. The NS1-HA2, blunt ended junction was sequenced as follows:

```
          7          1      2      3
5'  AAAATGACCATG  GATCTG  TCCAGA  GGT      3'
```

Region 7 is derived from the NS1 gene. Regions 1, 2 and 3 are defined above.


Example 3. Production of the C13 Protein


E. coli host strain N5151, a temperature sensitive defective lambda lysogen (cl857) was transformed with pC13. Transformants were grown at 32°C to mid-log phase ($A_{260}$ = 0.6) in LB broth supplemented wth 100 micrograms/ml of ampicillin. The cultures were then shifted to 42°C to inactivate cl and thus induce synthesis of the C13 protein. After 2 hours at 42°C, the bacteria were collected by centrifugation (3500 rpm, 20 min) and the bacterial pellet was frozen at -20°C.

The pellet was thawed and resuspended in buffer A (50 mM Tris-HC1, pH 8.0, 2Mm EDTA, 1mM dithiothreitol, 5% (vol/vol) glycerol). Lysozyme was added to a final concentration of 0.2 mg/ml, and the mixture was incubated on ice for 20 minutes. The mixture was then treated in a Warin blender at high speed for six bursts of 15 seconds each. The suspension was then sonicated for one minute with a reason probe sonifier. The mixture was then centrifuged (15,000 rpm, 30 minutes).

The pellet was resuspended in buffer A by sonication 4 x 15 second bursts). The mixture was then made 0.1% deoxycholate and stirred for one hour at 4°C. The mixture was centrifuged (15,000 rpm, 30 minutes) and the protein was pelleted. The deoxycholate treatment was repeated and the resulting pellet was then resuspended in buffer A by sonication. The suspension was then made 1% Triton X-100 and stirred for one hour at 4°C. The mixture was again centrifuged (15,000 rpm, 30 minutes) and the protein pellet was collected. The protein was resuspended by sonication and the protein was solubilized with urea (4M final concentration). This solution was centrifuged to remove any particulate material (15,000 rpm, 30 minutes) and the supernatant was collected and dialyzed against three, one liter changes of 10 mM Tris-HC1, pH 7.5, 1 mM EDTA to remove the urea. The protein solution was again centrifuged to remove any particulate material (15,000 rpm, 30 minutes) and the supernatant which contained the C13 protein, was collected and used for assays. The yield of C13 protein is about 10% of total cellular protein as determined by SDS-PAGE.


Example 4. Plasmid pD


Plasmid pAS1ΔEH/801 (described above in Example 1) was cut with BglII, end-filled with DNA polymerase I (DNApoll; Klenow) and ligated closed, thus eliminating the BglII site. The resulting plasmid pBgl⁻ was digested with NcoI, end-filled with DNApoll (Klenow) and ligated to a BglII linker. The resulting plasmid, pB4, contains a BglII site within the NS1 coding region. Plasmid pB4 was digested with BglII and ligated to a synthetic DNA linker of the sequence:

$$5'-GATCCCGGGTGACTGACTGA \quad . \quad -3'$$
$$3'- \quad GGCCCACTGACTGACTCTAG-5'$$

The resulting plasmid, pB4+, permits insertion of DNA fragments within the linker following the coding region for first 81 amino acids of NS1 followed by termination codons in all three reading frames. Plasmid pB4+ was digested with XmaI (cuts within linker), end-filled (Klenow) and ligated to a 520 base pair PvuII/HindIII, end-filled fragment derived from the HA2 coding region. The resulting plasmid, pD, codes for a protein comprised of the first 81 amino acids of NS1, three amino acids derived from the synthetic DNA linker (gln-ile-pro), followed by amino acids 65-222 of the HA2, as shown in Figure 2.

## Example 5. Plasmid pC13short

Plasmid pAS1ΔEH801 (described in Example 1) was cut with NcoI and SalI and ligated to a synthetic DNA encoding human TGFα as an NcoI/SalI fragment. The resulting plasmid, pNS1₈₁TGFα, encodes a protein comprised of the first 81 amino acids of NS1 and the mature TGFα sequence. Plasmid pNS1₈₁TGFα was cut with HindIII and NcoI to liberate a 218 base pair fragment encoding amino acids 8 to 81 of the NS1. A synthetic DNA encoding the first 42 amino acids 8 to 42 of NS1 (with the exception that amino acid #13 of the NS1 sequence was changed from a cysteine to a serine) as a HindIII/NcoI fragment was then ligated into the plasmid. The resulting plasmid, pNS1₄₂TGFα, encodes a protein comprised of the first 42 amino acids of NS1 and the mature TGFα sequence. Plasmid pNS1₄₂TGFα was then digested with NcoI and ligated to a 704 base pair NcoI fragment encoding the HA2 region derived from pC13. The resulting plasmid, "pC13 short", encodes a protein "C13 short", comprised of the first 42 amino acids of NS1 (with the exception of amino acid #13 changed from a cysteine to a serine), three amino acids encoded by a synthetic DNA linker (met-asp-leu), amino acid (326) from the carboxy terminus of the HA1 (ser), an arginine residue (327) separating the HA1 and HA2 subunits, and the entire HA2 subunit, as shown in Figure 3.

## Example 6. Plasmid pDshort

.Plasmid pMG27N a pASI derivative (Mol. Cell Bio. 5, 1015-1024 (1985)), was cut with BamHI and SacI and ligated to a BamHI/NcoI fragment encoding the first 81 amino acids of NS1 from pAS1ΔEH801 and a synthetic DNA NcoI/SacI fragment of the following sequence:

$$5'-CATGGATCATATGTTAACAGATATCAAGGCCTGACTGACTGAGAGCT-3'$$
$$3'- \quad CTAGTATACAATTGTCTATAGTTCCGGACTGACTGACTC \quad -5'$$

The resulting plasmid, pMGI, allows the insertion of DNA fragments after the first 81 amino acids of NS1 in either of the three reading frames within the synthetic linker fragment followed by termination codons in all three reading frames. To derive a similar vector which contains the coding region for the first 42 amino acids of NS1 rather than the first 81 amino acids of NS1, pMG1 was digested with BamHI and NcoI and ligated to the BamHI/NcoI fragment encoding amino acids 2 to 42 of NS1 from pNS1₄₂TGFα. The resulting plasmid, termed pMG42A, was then modified to contain an alternative synthetic linker after the NS1₄₂ sequence with a different set of restriction enzyme sites within which to insert foreign DNA fragments into the three reading frames after the NS1₄₂. This linker has the following sequence:

$$5'-CATGGATCATATGTTAACAAGTACTCGATATCAATGAGTGACTGAAGCT-3'$$
$$3'- \quad CTAGTATACAATTGTTCATGAGCTATAGTTACTCACTGACT \quad .-5'$$

The resulting plasmid, pMG42B was then digested with EcoRV which cuts within the synthetic linker and Xhoi. It was then ligated with a PvuII/SalI fragment derived from pMS₂ to generate plasmid "pD short".

This plasmid encodes a protein, "D short", which is comprised of the first 42 amino acids of NS1 (with the exception of amino acid #13 changed from a cysteine to a serine), 10 amino acids derived from the synthetic linker (met-asp-his-met-leu thr-ser-thr-arg-ser) and amino acids 66-222 of the HA2 subunit, as shown in Figure 4.

### Example 7. Plasmid pC13(H69-222)

Plasmid pB4+ (described in Example 4) was cut with XmaI, end-filled (Klenow) and a 508 base pair EcoRI/HindIII, end-filled fragment derived from the HA2 coding region was ligated to it. The resulting plasmid pC13(H69-222) codes for "A" protein comprised of the first 81 amino acids of NS1, three amino acids derived from the synthetic DNA linker (gln-ile-pro), and amino acids 69-222 of the HA2 subunit.

### Example 8. Plasmid pC13(H81-222)

Plasmid pB4+ (described in Example 4) was cut with SmaI and ligated to a 474 base pair AhaIII/HindIII, end-filled fragment derived from the HA2 coding region. The resulting plasmid, pC13(H81-222), codes for "C" protein comprised of the first 81 amino acids of NS1, three amino acids derived from the synthetic DNA linker (gln-ile-pro) and amino acids 81-222 of the HA2 subunit.

### Example 9. Plasmid pC13(HA150-222)

Plasmid pJZ102 (described in Example 2) was cut with HindIII to liberate the HA cDNA. This 1784 base pair fragment was isolated and ligated into pUC8 which had been cut with HindIII. The resulting plasmid, pMS2, was then cut with BsmI, mungbean nuclease-treated then SalI digested. A 280 base pair fragment containing the C-terminal coding region of the HA2 was then isolated. This fragment was ligated into pB4+ plasmid which had been cut with XmaI, end-filled (Klenow) then cut with SalI. The resulting plasmid, pC13-(H150-222), codes for a "ΔD" protein comprised of the first 81 amino acids of NS1, four amino acids encoded by the synthetic DNA linker (gln-ile-pro-val) followed by amino acids 150-222 of the HA2 subunit.

### Example 10. Plasmid pΔ13

Plasmid pC13 was cut with EcoRI to release a 1163 base pair fragment and ligated closed to yield plasmid pΔ13. This manipulation results in the loss of the coding region for the C-terminal 152 amino acids of the HA2. Consequently, the fusion protein "Δ13" produced from this plasmid contains the first 81 amino acids of the NS1, two amino acids encoded by a synthetic DNA linker (asp-leu), one amino acid from the carboxy terminus of the HA1 (ser), an arginine residue (327) separating HA1 from HA2, the first 70 amino acids of the HA2 followed by eight amino acids derived from the pBR322 sequence (ser-cys-leu-thr-ala-tyr-his-arg).

### Example 11. Plasmid pC13(H65-196)αMSH

Plasmid pMS2 (described in Example 9) was digested with BstXI and SalI and ligated to a synthetic linker encoding α-melanocyte stimulating hormone (αMSH). The resulting plasmid, pMS2αMSH, was digested with EcoRI (within the HA2 coding region) and SalI (at the carboxy terminus of the αMSH coding sequence) releasing a 431 base pair fragment which was isolated and ligated into plasmid pD that had been digested with EcoRI and SalI. The resulting plasmid, pC13(H65-196)αMSH, encodes a hybrid protein "M" comprised of the first 81 amino acids of NS1, three amino acids derived from the synthetic DNA linker (gln-ile-pro), amino acids 65-196 of the HA2 subunit, two glycines, followed by αMSH (ser-tyr-ser-met-glu-his-phe-arg-trp-gly-lys-pro-val).


## Example 12. Plasmid pC13(H65-196)ΔMSH


Plasmid pC13(H65-196)αMSH was digested with NcoI, end-filled (Klenow) and ligated to a 12 base pair translation stop linker of the sequence:

5'-CTAGTTAACTAG-3'
3'-GATCAATTGATC-5'

The resulting plasmid, pC13(H65-196)AMSH, encodes "ΔM" protein comprised of the first 81 amino acids of NS1, three amino acids derived from the synthetic DNA linker (gln-ile-pro), amino acids 65-196 of the HA2 subunit, gly-gly, the first 4 amino acids of αMSH (ser-tyr-ser-met) followed by three amino acids derived from the termination linker (leu-val-asn).


## Example 13. Plasmid pC13(H65-200)


Plasmid pMS2 (described in Example 9) was digested with BstXI and SalI and ligated to a synthetic linker having the sequence:


$$\textbf{5' \quad CTGGTGCTTTTGTAG \quad 3'}$$
$$\textbf{3' \quad AAGTGACCACGAAAACATCAGCT \quad 5'}$$


The resulting plasmid, pMS2-anchorless, was digested with EcoRI (within the HA2 coding region) and SalI, releasing a 331 base pair fragment which was isolated and ligated into plasmid pD that had been digested with EcoRI and SalI. The resulting plasmid, pC13(H65-200), encodes a hybrid protein "ΔM+" comprised of the first 81 amino acids of NS1; three amino acids derived from the synthetic DNA linker (gln-ile-pro) (described above in Example 4), amino acids 65-196 of the HA2 subunit, followed by leu-val-leu-leu derived from the synthetic linker described immediately above which restores amino acids corresponding to amino acids 197-200 of the HA2 subunit.


## Example 14. Purification of D Protein


Following the induction of synthesis of D protein by an E. coli host strain, the bacterial cells were collected by centrifugation and the resulting pellet frozen at -70 C. The pellet was thawed and resuspended in lysis buffer A (50 mM Tris, 2mM EDTA, 0.1 mM dithiothreitol (DTT), 5% glycerol, at pH 8) by adding 10 mls of lysis buffer A for each gram of cell paste, and thawing at room temperature. To the resulting suspension was added a concentrated lysozyme solution to give a final concentration of at least about 0.2 mg/ml lysozyme. The suspension was stirred at room temperature for about 1 to 1.5 hour and then lysed on a Manton Gaulin homogenizer (APV Gaulin, Inc., Everett, Massachusetts) (10,000 psi) in two passes. To this suspension was added Triton X-100 to a final concentration of 1% and deoxycholate added to a final concentration of 0.1%. The suspension was stirred at room temperature for 1 hour and centrifuged at 25,000 Xg for about 1 hour. The supernatant was discarded and the pellet containing the protein was suspended in glycine buffer (50 mM gly-NaOH + 2 mM EDTA +5% glycerol) (pH 10.5), 10 ml buffer/gram of initial cell paste, using a Turrax homogenizer, until all lumps were gone. To this suspension was added Triton X-100 to a final concentration of 1%, bringing the final volume to 10 ml for every 1 gram of initial cell paste. The suspension was stirred at 4 C for 1 hour, centrifuged (25,000 Xg, 1 hour) and the supernatant discarded. The pellet containing D protein was dissolved in 8 M urea + 50 mM Tris (pH 8.0) at room temperature for 1 to 2 hours, then overnight at 4 C followed by centrifugation (25,000 xG, 1 hour) to remove insoluble contaminants. D protein remained in the supernatant. To the supernatant was added DTT to a final concentration of 50 mM and the solution stirred for about 1 hr. at room temperature. The stirred solution was then loaded onto a DEAE Fast Flow Sepharose column equilibrated with a solution of 8M urea and 50 mM Tris (pH 8). A maximum load ratio of 4 mg protein/ml gel and a minimum of 22 cm column length was maintained in this step. D protein was eluted with a 0 to 0.3 M NaCl gradient (over five column volumes or greater) in 8M urea, 50 mM Tris (pH 8). Purified D protein eluted in a broad peak centering around 0.1 M NaCl. Fractions containing the protein were dialyzed into a solution of 20mM Tris and 2mM EDTA at pH 8.

The yield of D protein was about 23% of total cellular protein as determined by laser densitometric analysis of reducing SDS-PAGE gels.

C13, C13 short, D short, A, C, $\Delta$D, $\Delta$M and $\Delta$M+ proteins are similarly purifiable by the method herein described for the purification of D protein.

## Example 15. Further Purification of D Protein

Following the elution of D protein-containing fractions from the DEAE Fast Flow Sepharose column described above in Example 14, the fractions were concentrated 15-fold (3.8 liters to 255 ml) on a Minisette tangential flow apparatus (Pharmacia) equipped with a 500 $cm^2$ Omega 10 membrane and screen channel, operated at 15-20 pounds per square inch transmembrane pressure at a cross flow rate of 1000 ml per minute. To the concentrate was added a 10-fold excess of sodium dodecyl sulfate (SDS) (Sigma Chemical Co., St. Louis, MO. U.S.A.), that is, 10 mg SDS per mg of protein, and dithiothreitol (DTT) (Sigma Chemical Co.) to a final concentration of 50 mM. The solution was stirred at room temperature for ninety minutes and then loaded (16 cm per hour) onto a 2800 ml Superose 12 column (Pharmacia) equilibrated with a buffer (pH 8.0) containing 25 mM Tris Glycine and 1% SDS. The protein was eluted isocratically with the column equilibration buffer. Those fraction.s of adequate purity, as determined by SDS-PAGE and Western Blot analyses, were pooled.

The pooled fractions described above were concentrated on a Minisette tangential flow apparatus described above. To the concentrated fractions was added a ten fold excess of SDS (10 mg SDS per mg protein) and DTT to a final concentration of 50 mM. The resulting solution was stirred for ninety minutes at room temperature. The solution was then loaded (16 cm per hour) onto a 2800 ml Superose 12 column and chromatographed under the same conditions as described immediately above. Eluted fractions were analyzed by SDS-PAGE and Western Blot analyses for purity. Those fractions of adequate purity were pooled.

To remove SDS from the pooled fractions, the fractions were first concentrated on an Omega 10 stirred cell apparatus (Pharmacia). The concentrated sample was then loaded (25 cm per hour) onto a 1443 ml G25 Sephadex fine chromatography column (Pharmacia) (4.4 X 95 cm) previously equilibrated with a buffer (pH 8) containing 50 mM Tris and 8 M urea. The column was eluted isocratically at the same flow rate with the equilibration buffer. Fractions were collected, assayed for protein and SDS levels and then pooled to maximize recovery of the protein free of SDS contamination.

Following SDS removal, the purified protein (approximately 90 % pure and virtually free of endotoxin) was dyalized against a buffer (pH 8) containing 20 mM Tris and 1 mM EDTA. Following dialysis, the sample was sterile filtered and stored at 4° C. To lyophilize the protein, the sample was dialyzed against 20 mM sodium bicarbonate, frozen in a dry ice ethanol bath and then lyophilized. Lyophilized protein was then reconstituted with a buffer (pH 8) containing 20 mM Tris and 1 mM EDTA.

## Example 16. T Cell Assay

The ability of the C13 protein to induce a cytoxic T cell response in an in vitro assay was compared to that of other proteins also of A/PR/8/34 origin. The other proteins are herein identified as:

| C7 | (complete HA) |
| Delta 7 | (HA1 and 80 N terminal residues of HA2) |
| C36 | (HA2) |
| Delta 13 | (80 N terminal residues of NS1 and 80 N terminal residues of HA2) |
| NS1 | (NS1) |
| NS2 | (NS2) |
| M30 | (NS1 and M) (See Example 1) |

Molecules comprising the coding sequence for each of these were derived as described by Young et al., in The Origin of Pandemic Influenza Viruses, 1983, edit. by W.G. Laver, Elsevier Science Publishing Co. and were expressed in pAS1deltaEH substantially as described above. Except for NS1, the proteins were produced substantially as described in Example 3. Following resuspension of the bacterial pellet, lysozyme treatment, sonication and centrifugation, the NS1 protein was contained in the supernatant rather than in the pellet. Recovery of NS1 was completed as described below.

The supernatant was brought to 100 mM $MgCl_2$ and stirred for one hour at 4° C. The solution was then centrifuged (15,000 rpm, 30 minutes) to pellet the NS1. The pellet was resuspended in buffer A (described in Example 3) and again brought to 100 mM $MgCl_2$ to reprecipitate the NS1 protein. Following a recentrifugation, the pellet was resuspended in buffer A and dialyzed against three one-liter changes of 10 mM Tris-HC1, pH 7.5, 1 mM EDTA. The solution was then centrifuged again to remove any particulate material and the supernatant containing the NS1 protein was collected and used for assays.

The cytoxic T cell assay was carried out substantially as follows. Spleen cells were isolated from virus-immune or non-immune mice and cultured in vitro. Cells were subdivided and exposed to various antigens for 90 mins in vitro. The antigens were then removed by repeated washing of cells and the cells were then cultured for 5 days to allow expansion of stimulated populations. Stimulated cells, that is, effector cells, were mixed with virus-infected or un-infected P815 (mouse mastocytoma) target cells each of which had been pre loaded with $^{51}Cr$. Significant release of $^{51}Cr$ into the culture medium indicated a presence of secondary cytotoxic T cells (2° CTL), which had been generated by the in vitro stimulation with antigen. The specificity of killing was examined in two ways: 1) target cells infected with different viruses were tested for killing; and, 2) spleen cells were isolated from mice which had been immunized with different viruses. The linearity of the assay was also examined using different target:effector cell ratios and using different amounts of antigen, as indicated in the following table, which shows illustrative results. Results, listed below, indicate effector:target cell ratios which were 30:1 ("30") and 10:1 ("10").

Values in the tables are expressed as the percent of $^{51}Cr$ released into the medium compared to the total amount of $^{51}Cr$ in cells as determined by detergent solubilization of cells. Significant positive results are enclosed in boxes. Viruses used in the assays were:

| A/PR/8/34 (H1N1) | ("PR8") |
| A/Port Chalmers/1/74 (H3N2) | ("A/PC") |
| A/Brazil/1/78 (H2N1) | ("A/BZ") |
| A/Singapore/1/57 (H2N2) | ("A/Sing"). |

TABLE 1

| STIMULATION OF 2° CTL RESPONSE BY E. COLI-DERIVED POLYPEPTIDES | | | | | |
|---|---|---|---|---|---|
| Antigen used for 2° Stimulation | | PR8-P815 | | Uninfected-P815 | |
| | | 30 | 10 | 30 | 10 |
| PR8 | | 36.0 | 27.2 | 0.2 | -5.2 |
| C13 | 24μg/ml | 10.7 | 10.7 | 0.5 | -4.4 |
| | 12μg/ml | 10.6 | 8.1 | 0.5 | -3.6 |
| | 6μg/ml | 9.5 | 4.4 | 1.4 | -4.1 |
| NS₂ | 24μg/ml | 0.4 | 2.8 | -4.0 | -2.7 |
| | 12μg/ml | -1.8 | -1.5 | 0.2 | -4.1 |
| | 6μg/ml | -0.5 | -2.0 | -0.3 | -5.2 |
| M30 | 24μg/ml | -1.0 | -3.9 | -1.6 | -3.3 |
| | 12μg/ml | -1.8 | -1.2 | -1.9 | 1.1 |
| | 6μg/ml | 5.7 | -1.5 | -3.6 | -6.2 |
| None | | -2.7 | -4.1 | -4.7 | -4.0 |

TABLE 2

| STIMULATION OF 2° CTL RESPONSE BY E. COLI-DERIVED POLYPEPTIDES* | | | | |
|---|---|---|---|---|
| Antigen used for 2° Stimulation | PR8-P815 | | Uninfected-P815 | |
| | 30 | 10 | 30 | 10 |
| PR8** | 60.5 | 37.4 | 6.7 | 2.6 |
| NS₁ | -5.2 | -8.7 | 4.3 | 0.0 |
| C13 | 15.0 | -1.1 | 0.5 | -0.5 |
| Δ13 | -8.6 | -10.1 | -1.3 | 0.0 |
| Δ7 | -3.2 | -7.4 | 2.9 | -0.9 |
| None | -5.4 | -2.0 | 1.9 | 3.2 |

* Spleen cells taken from PR8-immune mice were stimulated in vitro with antigens (5μg/ml).
** PR8-infected syngeneic spleen cells.

## TABLE 3

### VIRUS SPECIFICITY OF CTL STIMULATED BY POLYPEPTIDE C13

| Effector | | PR8–P815 | | A/PC–P815 | | Uninfected–P815 | |
|---|---|---|---|---|---|---|---|
| 1•* | ·2• | 30 | 10 | 30 | 10 | 30 | 10 |
| PR8 | PR8 | 80.5 | 59.4 | 72.8 | 55.8 | 4.2 | 0.2 |
| | A/PC | 77.9 | 76.1 | 74.2 | 59.3 | 2.3 | 2.1 |
| | C13 24µg/ml | 33.2 | 14.8 | -0.4 | -0.7 | 1.7 | 1.3 |
| | 12µg/ml | 11.0 | 3.9 | 2.4 | 2.4 | 4.4 | -1.4 |
| | 6µg/ml | 8.0 | 1.2 | 2.3 | 2.3 | 0.8 | 0.4 |
| A/PC | PR8 | 96.0 | 72.9 | 74.3 | 57.5 | 9.8 | 1.8 |
| | A/PC | 92.3 | 75.6 | 85.1 | 80.0 | 10.2 | 3.8 |
| | C13 24µg/ml | 6.7 | 1.0 | 4.3 | -5.0 | 2.6 | 0.2 |
| | 12µg/ml | 4.6 | -1.2 | 1.2 | -3.7 | 2.4 | -0.3 |
| | 6µg/ml | 5.2 | 1.5 | -0.2 | -6.3 | 3.1 | -1.5 |

* Spleen cells were isolated from mice which had been pre-immunized with the indicated virus

EP 0 366 239 A1

## TABLE 4

### VIRUS SPECIFICITY OF CTL STIMULATED BY POLYPEPTIDE C13

| Effector 1 | 2 | A/PR/8(H1N1) 30 | 10 | A/BZ 30 | 10 | A/SING 30 | 10 | A/PC 30 | 10 | Uninfected 30 | 10 |
|---|---|---|---|---|---|---|---|---|---|---|---|
| PR8 | PR8 | 76.5 | 77.0 | 82.8 | 75.5 | 30.6 | 17.8 | 92.8 | 79.0 | 8.4 | 3.0 |
| | C13 48μg/ml | 60.9 | 42.6 | 65.5 | 41.3 | 5.8 | 0.0 | 5.7 | 7.5 | 4.1 | 3.0 |
| | 24μg/ml | 70.1 | 46.5 | 63.5 | 46.2 | 9.5 | 5.6 | 9.2 | 9.3 | 8.8 | 0.6 |
| | 12μg/ml | 50.7 | 24.0 | 45.0 | 18.4 | 2.0 | 4.4 | 10.3 | 8.5 | 2.0 | 1.1 |
| None | PR8 | 10.6 | -0.8 | 14.0 | 16.3 | 15.3 | 6.7 | 13.2 | 5.6 | 2.0 | 1.5 |
| | C13 48μg/ml | -0.8 | -0.6 | 8.4 | 3.6 | 4.6 | 3.7 | 2.5 | 2.3 | 2.3 | -2.9 |
| | 24μg/ml | 0.7 | 0.3 | 7.6 | 7.3 | 5.2 | 6.7 | 4.8 | 4.6 | 5.1 | -2.0 |
| | 12μg/ml | 1.8 | -0.9 | 10.9 | 2.9 | 3.6 | 2.5 | 4.9 | 2.9 | 5.1 | 8.3 |

EP 0 366 239 A1

As indicated in the preceding tables, C13 induces a secondary cytotoxic T cell response using immune spleen cells from mice previously infected with sublethal doses of PR8 virus. All other peptide derivatives that were studied, including NS1, delta7, delta13, M30, NS2 and C36, failed to induce such a response, as have certain hemagglutinin constructs. The response to the C13 peptide is dose dependent and levels from 6 micrograms per ml through 24 micrograms per ml induced secondary cytotoxic T cell responses.

The viral specificity of the observed responses is striking in that C13 stimulated immune spleen cells from mice previously infected with H1N1 virus but did not stimulate immune spleen cells in mice previously infected with H3N2 virus (A/PC). This is unlike the subtype cross-reactive cytotoxic T lymphocyte responses observed when stimulating the same spleen cells with live virus due, at least in part, to the cross-reactive internal antigens. In addition, the stimulation by C13 is cross-reactive among virus strains in the H1N1 subtype; PR8 immune spleen cells stimulated by C13 in vitro were able to recognize and kill target cells infected with PR8 (H1N1 strain from 1934) as well as target cells infected with the A/Brazil (H1N1 strain of 1978) over a dosage range of 12 micrograms through 48 micrograms and at a high degree of cytotoxic activity.

Based on substantial data showing that cytotoxic T lymphocytes appear to contribute to recovery from influenza virus infection in mouse systems and that such lymphocyte responses can be detected in both immune mice and humans (see, Ennis et al., Microbiology - 1984, pages 427-430, Amer. Soc. Microbiology), the ability of the C13 protein to induce such cytoxic T cells response across strains indicates its utility, and the utility of the HA2 immunogenic determinant, to induce an immune response which will resist influenza virus infection; the response is semi-universal in that it is subtype, but not strain, specific.

### Example 17. C13 T Cell Assay and Protection Studies

This Example describes three studies in which BALB/c mice were immunized with C13 protein demonstrating that immunization induces protection against influenza infection via induction of CTL.

### First Study: Virus Specificity of C13 Immune Spleen Cells

In the first study, four week old male BALB/c mice were given 300 ug of C13 protein intraperitoneally and repeat doses 3, 4 and 5 weeks later. (Prior experiments had indicated that complete Freund's adjuvant did not significantly enhance the level of C13 protein-induced CTL activity). One week after the fourth immunization, spleen cells of mice were isolated and cultured for in vitro stimulation with virus. Non-immunized mice (controls) were infected intranasally with 100 plaque forming units (PFU) of A/PR/8 virus 4 weeks prior to removal of spleen cells for in vitro secondary stimulation. CTL assays were carried out substantially as described in Example 4, above. Briefly, $3 \times 10^7$ spleen cells from immunized or control mice were cultured with $3 \times 10^6$ syngeneic normal spleen cells which had been infected with A/PR/8 or A/PC virus at a multiplicity of infection of 10 PFU per cell. After five days of culture these cells were used as effector cells. For target cells, $2 \times 10^6$ P815 cells were incubated with A/PR/8 or A/PC virus at a multiplicity of infection of 10 PFU per cell in the presence of 250 uCi of $^{51}Cr$, and $1 \times 10^4$ $^{51}CR$-labeled target cells were incubated with effector cells at the indicated ratios in a 96-well round-bottom microplate for 4 hours. The supernatant fluids were harvested and $^{51}Cr$ was measured. Percent specific lysis was determined as follows: percent specific lysis = (experimental release - minimum release) x 100/(maximum release - minimum release) where spontaneous release was determined by incubating P815 cells in medium, and maximum release by incubating P815 cells in 10% Renex 30 solution (Ruger Chem. Co., NJ). E:T (effector/target) ratios varied from 3:1 ("3") to 200:1 ("200") as listed, and quadruplicate samples were tested at each E:T ratio. Results are shown in Table 5, below.

17

Table 5

| Virus Specificity of C13 Immune Spleen Cells Stimulated by A/PR/8 (H1N1) or A/PC (H3N2) Viruses | | | | | | | | | | |
|---|---|---|---|---|---|---|---|---|---|---|
| STIMULATION | | PERCENT SPECIFIC LYSIS | | | | | | | | |
| ( in vivo ) | ( in vitro ) | A/PR/8 (H1N1) | | | A/PC (H3N2) | | | Uninfected | | |
| 1°-4° | 5° | 200 | 60 | 20 | 200 | 60 | 20 | 200 | 60 | 20 |
| c13 | A/PR/8 | 30 | 13 | 5 | 1 | 0 | 2 | -3 | -3 | -3 |
| | A/PC | -1 | 3 | 3 | 0 | -1 | 0 | 1 | 0 | 3 |
| | None | 2 | 0 | -1 | 0 | -1 | -2 | -4 | -3 | -3 |
| None | A/PR/8 | 0 | -2 | -1 | -1 | -1 | -1 | -4 | -4 | -3 |
| | A/PC | -1 | -1 | 0 | 1 | 1 | 0 | -1 | -3 | -1 |
| | None | 0 | 0 | 2 | -1 | 0 | 2 | -4 | -4 | -2 |
| ( in vivo ) | ( in vitro ) | A/PR/8 (H1N1) | | | A/PC (H3N2) | | | Uninfected | | |
| 1° | 2° | 25 | 8 | 3 | 25 | 8 | 3 | 25 | 8 | 3 |
| A/PR/8 | A/PR/8 | 65 | 32 | 11 | 59 | 31 | 20 | 4 | 6 | 0 |
| | A/PC | 32 | 10 | 8 | 34 | 16 | 8 | -3 | -4 | -1 |
| | None | -1 | -2 | -3 | 0 | -1 | -1 | -3 | -4 | -3 |

Table 5 shows that the spleen cells of C13 immunized mice which were stimulated by A/PR/8 virus-infected normal syngeneic spleen cells in vitro were able to lyse A/PR/8 infected target cells, although to a lower degree than A/PR/8-immune effector cells stimulated by A/PR/8 infected normal spleen cells, and they did not lyse A/PC (H3N2) virus infected target cells or uninfected target cells. No detectable CTL activity was found in C13 protein-immune spleen cells after stimulation by A/PC (H3N2) virus. Spleen cells of non-immune mice also failed to show CTL activity on any target cells after stimulation with virus in vitro.

### Second Study: Pulmonary Virus Titers of C13 Immunized Mice

In the second study, the pulmonary virus titers of C13 protein immunized mice and non-immune mice were examined after challenge with A/PR/8 (H1N1) or A/PC (H3N2) viruses. One week after the fourth immunization (as in Table 5), mice were intranasally challenged under ether anesthesia with A/PR/8 or A/PC virus at a dose of 5 X $10^4$ PFU. Four days later the lungs were aseptically harvested for measurement of pulmonary virus titer. Harvested lungs were manually homogenized in 1.5 ml of PBS followed by centrifugation (2000g. for 15 min at 4° C). Supernatants were frozen until they were titrated for virus. Madin Darby canine kidney (MDCK) cells were maintained in Eagle Minimum Essential Medium (MEM) containing 100 ug/ml penicillin, 100 ug/ml streptomycin and 200 ug/ml L-glutamine supplemented with 10% heat-inactivated fetal bovine serum, and were seeded (25 X $10^4$ cells in 1 mg of MEM) in 24 well tissue culture plates. Lung supernatants were thawed and serially diluted in PBS containing 0.1% bovine albumin. After aspiration of medium from the wells, 100 ul of the diluted virus solution was added to each well and incubated at 37° C for 1 h with occasional agitation. Each well then received 1 ml of agar medium containing MEM, 0.1% D glucose, 0.01% DEAE-Dextran, 1% vitamins (16-004-49; Flow Laboratories, McClean, VA, USA), 10 ug/ml trypsin and 1% agar. After 2 days incubation in 5% $CO_2$ at 37° C, 1 ml of 10% neutral red in PBS was overlaid in agar in each well. Plaques were counted after 10 h incubation. The results are expressed as the mean $\log_{10}$ PFU/ml of duplicate samples in Table 6, below.

Table 6

| Pulmonary Virus Titer of C-13 Immunized Mice | | |
|---|---|---|
| IMMUNIZATION | RECIPIENTS | |
| VIRUS CHALLENGE | VIRUS TITER | |
| c13 | A/PR/8 (H1N1) | 3.8 ± 0.9* |
| None | A/PR/8 | 5.4 ± 0.2* |
| c13 | A/PC (H3N2) | 5.4 ± 0.2 |
| None | A/PC | 5.0 ± 0.3 |

*P<0.005, determined by Student's t-test.

Table 6 shows that the C13-immunized mice had significantly lower pulmonary virus titers of A/PR/8 virus compared to virus titers in non-immunized mice. There was no significant difference in the pulmonary virus titers between C13 immunized mice and non-immune mice after A/PC (H3N2) virus-challenge.

After A/PR/8-lethal challenge infection seven out of eight C13-immunized mice survived beyond day 60 (last day of observation), but all non-immune mice were dead by day 7. This protection induced by C13 protein reflects the specificity of lysis by the virus-stimulated C13 immune spleen cells of A/PR/8-infected target cells in vitro, and the specific limitation of A/PR/8 virus replication in the lungs of mice which had been immunized by C13 protein.

### Third Study: C13 Immunized Mice Challenged with A/TAIWAN/1/86

In the third study, protection of C13 protein immune mice against a recently isolated H1 subtype virus strain, A/Taiwan/1/86 (H1N1) was also examined. An additional 200 ug dose of C13 protein was given 3 weeks after the fourth immunization (Table 5). One week later mice were challenged intranasally with the A/Taiwan/1/86 (A/TW/1/86) virus strain (obtained from the Office of Biologics, U.S. Food and Drug Administration), at a dose of 1 X 10$^5$ PFU under ether anesthesia. Before challenge, serum was obtained from C13 protein-immunized mice for titration of neutralizing antibody. Four days after infection, lungs of mice were removed for virus titrations as described for Table 6. Neutralizing antibody against A/PR/8 virus was determined by a plaque assay in MDCK cells. Serially diluted pooled sera were preincubated with 20 PFU of virus at 37°C for 1 h and were titrated for virus, as for Table 6. The 50% plaque neutralizing antibody titers were calculated. Pooled sera of mice which had been infected intranasally six weeks earlier with 100 PFU of A/PR/8 virus were included as a positive control. Results are reported in Table 7, below.

Table 7

| Pulmonary Virus Titer of C13 Immunized Mice | | | |
|---|---|---|---|
| | RECIPIENTS | | |
| IMMUNIZATION | VIRUS CHALLENGE | VIRUS TITER | NEUTRALIZING Ab Titer |
| C13 | A/TW/1/86 (H1N1) | 2.5 ± 0.9* | <4 |
| None | A/TW/1/86 (H1N1) | 4.1 ± 0.3* | <4 |
| A/PR/8 | N.D. | N.D. | 256 |

*P<0.005, determined by Student's t-test.

The results shown in Table 7 demonstrate that the C13 protein-immunized mice had significantly lower lung virus titers of A/Taiwan/1/86 after challenge than did non-immune mice; however, neither the C13 protein-immunized nor control non-immune mice had serum neutralizing antibodies against A/PR/8 virus. This was expected because the HA2 subunit does not contain sites which induce neutralizing antibodies. In contrast, A/PR/8 virus-primed mice, sampled as positive controls, showed high levels of neutralizing antibody titers. These results suggest that immunization with C13 protein induced protection against virus strains of the H1 subtype from 1934 and 1986 through the induction of CTL responses, and not by the induction of neutralizing antibody. These results demonstrate that immunization with a fusion protein which contains the HA2 subunit of influenza A (A/PR/8) virus induced protection against influenza A virus strains isolated over 50 years apart which have great diversity in hemagglutinin antibody specificities.

Example 18. Influenza Virus Specificity of D Protein Stimulated CTL

To assess the virus specificity of the D protein-stimulated CTL at the clonal level, D protein-stimulated virus immune spleen cells were cultured for eight weeks in the presence of irradiated syngeneic spleen cells in 10% supernatant fluid from Con A stimulated rat spleen cells. A limiting dilution of this D protein-stimulated CTL line was performed to develop CTL clones, all as more fully described below.

D protein was produced in recombinant E. coli substantially in the manner described for C13 protein in Example 3, above. Briefly, after lysis of the bacteria, two 0.1% deoxycholate extractions and one 1% Triton X-100 extraction were performed to remove contaminating E. coli proteins, and the D protein was solubilized with 4M urea at 4°C for 30 min. The urea was then removed by dialysis at 4°C. The prepared protein was stored in 50 mM Tris-HC1, pH 8.0, 1 mM EDTA.

Male BALB/c mice, 4-5 weeks of age, were immunized with 100 PFU of the virus intranasally under ether-anesthesia. The spleens of mice immunized with A/PR/8 were removed 3 or more weeks after immunization for in vitro stimulation.

Rat interleukin-2 (IL2) was prepared substantially as described by Townsend et al., J. Exp. Med. 160:552 (1984). Briefly, spleen cells from Lewis rats at the age of 2 months were adjusted to $2 \times 10^7$ lymphocytes/ml without lysing red blood cells and were incubated with Con A (Sigma Type III) (Sigma Chemical Co., St. Louis, MO) at a concentration of 20 ug/ml at 37°C for 2 h. Spleen cells were washed 3 times with PBS and cultured at $5 \times 10^6$ cells/ml in RPMI 1640 supplemented with 10% fetal bovine serum (FBS) at 37°C for 48 h. The supernatant fluid was harvested and frozen at -80°C after passing through a 0.45 um filter.

Secondary CTL were prepared substantially as described in Examples 16 and 17. To stimulate virus immune-spleen cells with D protein, spleen cells were cultured with D protein at a concentration of 50 ug/ml for 1 h, and were then washed twice with medium. After 5 days of culture they were used as effector cells in CTL assays.

CTL clones were established as follows. Viable secondary CTL in bulk culture were separated on Ficoll-Paque (Pharmacia, Piscataway, NJ). Syngeneic gamma-irradiated (2000 rad) spleen cells from non-immune BALB/c mice were pulsed with D protein (100 ug/ml) for 1 h at 37°C. Viable recovered cells ($20 \times 10^4$ cells/ml) were cultured with D protein pulsed gamma-irradiated spleen cells ($300 \times 10^4$ cells/ml) in 10 ml of medium in the presence of 10% (vol/vol) crude rat IL2 and $5 \times 10^{-5}$M 2 mercaptoethanol (2ME). This procedure was performed weekly to stimulate a CTL line. After 8 weeks of culture of this CTL line, a limiting dilution was carried out to develop CTL clones substantially as described by Braciale, et al., J. Exp. Med 153:910 (1981). Briefly, the medium used in this procedure was RPMI 1640 supplemented with 10% FBS, antibiotics (100 U/ml penicillin and 100 µg/ml streptomycin), $5 \times 10^{-5}$M 2-mercaptoethanol (2ME) and 10% Con A-induced rat IL2. Viable responder cells (0.5, 1, 2, 4, 8 and 16 cells/well) were cultured in wells of 96-well flat bottom microtiter plates (Costar, Cambridge, MA) with $1 \times 10^6$ syngeneic, D protein pulsed, irradiated BALB/c spleen cell in 0.2 ml of medium. Stimulator cells were added every 7 days. As clones grew, they were expanded in larger vessels such as 24 well tissue culture plates (Costar), 6 well culture plates (Falcon, Oxnard, CA) or 25 cm² tissue culture flasks (Corning, Corning, NY).

P815 cells were used as target cells in $^{51}$Cr-release assays, which were carried out substantially as described above in Examples 16 and 17.

Two clones, designated H-6 and B-7, were established from the culture of D protein-stimulated virus-immune spleen cells described above; clone H-6 grew from a well in which two responder cells had been seeded and clone B-7 grew from a well in which 4 cells had been seeded. The cell surface phenotype of these clones are Thy 1.2[+] and Lyt 2[+]. These CTL clones are restricted by the H-2$^d$ haplotype, because they lyse A/PR/8 infected P815 (H-2$^d$) cells, but not A/PR/8 infected MC57G cells (H-2$^b$) or A/PR/8 infected BW5174 cells (H-2$^k$).

As shown in Table 8 below, these CTL clones exhibit cross-reactive cytotoxic activity against target cells infected with virus strains of the H1N1 (A/PR/8 and A/BZ) subtype and H2N2 (A/JAP) subtype; however, they failed to lyse target cells infected with H3N2 (A/PC) subtype virus or influenza B (B/HK) virus. Thus, these clones demonstrate cross-reactive lysis of target cells infected with H1 or H2 subtype viruses. This is different from the specificity of the secondary CTL observed in bulk cultures where the predominant killing is detected on target cells infected with virus of the H1 subtype, and little or no lysis is observed on target cells infected with virus strains of the H2 subtype. Such cross-subtype response was unexpected because it indicates that the vaccine of the invention can confer protection not only against all strains within a subtype but also across, at least certain, subtypes.

## Table 8

| Virus Specificity of CTL Clones in Vitro | | | | | | | |
|---|---|---|---|---|---|---|---|
| Percent specific $^{51}$Cr release from target cells | | | | | | | |
| CTL Clone | E/T ratio | P815 (H-2[d]) | | | | | |
| | | A/PR8 (H1N1) | A/BZ (H1N1) | A/JAP (H2N2) | A/PC (H3N2) | B/HK | Uninfected |
| H-6[a] | 1.0 | 56 | 49 | 53 | 10 | 3 | -3 |
| | 0.5 | 38 | 33 | 35 | 2 | 3 | 1 |
| B-7[b] | 1.0 | 65 | 68 | 42 | 0 | 2 | 0 |
| | 0.5 | 47 | 56 | 30 | -1 | 0 | -1 |

[a]Clone H-6 expresses 94% of Thy 1.2, 86% of Lyt2 and 5% of L3T4 surface antigens.
[b]Clone B-7 expresses 97% of Thy 1.2, 95% of Lyt2 and 0% of L3T4 surface antigens.

## In Vivo Effector Function of the H-6 CTL Clone

The H-6 CTL clone was then adoptively transferred to mice. Cells (2 X 10⁶) suspended in 0.5 ml RPMI 1640 were transferred intravenously via the tail vein into mice 6 hours before virus challenge. The lungs of infected mice were removed 3 days later and were homogenized to determine virus titers by plaque formation substantially as described in Example 17, above.

Table 9 below, shows that adoptive transfer of this CTL clone significantly reduced the virus titers in the lungs of mice infected with virus strains of the H1 (A/PR/8) and H2 (A/JAP) subtypes, but not those infected with the H3 (A/PC) subtype or type B influenza virus. These results indicate that this CTL clone which had been stimulated by the D protein and is specific for the HA2 subunit of H1 and H2 viruses, conferred protection in vivo. This confirms the predictive value of the in vitro CTL activity of the clone against target cells infected with H1 or H2 viruses.

## Cold Target Inhibition Study

To assure that the antigen specificity of D protein is equivalent to that of C13, cold-target inhibition experiments were performed using the D-protein stimulated B-7 CTL clone. Lysis of A/PR/8-infected target cells was inhibited by both C13 and D protein-coated cold target cells, as well as by A/PR/8 virus-infected cold target cells. Inhibition of lysis of C13 protein-coated $^{51}$Cr-labeled target cells was observed with cold A/PR/8 virus-infected C13 or D protein coated target cells. In both cases, neither A/PC-infected cold targets nor uninfected cold-target cells competed with the $^{51}$Cr labeled target cells. These results indicate that the D protein has the same antigenic specificity as C13 protein.

TABLE 9

| Virus specificity of pulmonary virus reduction in vivo by CTL clone H-6 | | |
|---|---|---|
| CTL Clone H-6 transferred[a] | Recipients | |
| | Virus challenge | Virus titer in lungs[b] |
| + | A/PR/8 (H1N1) | $5.0 \pm 1.0$ |
| - | A/PR/8 | $6.9 \pm 0.1^c$ |
| + | A/JAP (H2N2) | $2.6 \pm 0.3$ |
| - | A/JAP | $3.7 \pm 0.4^d$ |
| + | A/PC (H3N2) | $4.2 \pm 0.3$ |
| - | A/PC | $4.1 \pm 0.1$ |
| + | B/HK | $4.2 \pm 0.4$ |
| - | B/HK | $4.7 \pm 0.5$ |

[a] $2 \times 10^6$ cells were transferred 6 hours before virus challenge.
[b] 3 days after virus challenge, lungs were taken and virus titers were examined by plaque assay in MDCK cells.
[c] $P < 0.05$ determined by Student's T-test.
[d] $p < 0.02$ determined by Student's T-test.

## Example 19. Additional Peptides

Recombinant peptides additional to D protein were produced in recombinant E. coli and tested in the CTL assays substantially as described above. These peptides are listed in Table 10 below. Table 10 also lists the number of the first and last HA2 amino acids in the antigen and whether or not the antigen was positive ( + ) or negative (-) in the T cell assays. C13 and D protein are listed for reference. Amino acids are designated by one-letter symbols. Biochemistry 2nd Ed., A.L. Lehninger (1977).

Table 10

| C13, D protein and Derivatives | | CTL Activity |
|---|---|---|
| Protein | | |
| C13 | NS1$_{(1-81)}$-D-L-S-R-HA2$_{(1-222)}$ | + |
| D | NS1$_{(1-81)}$-Q-I-P-HA2$_{(65-222)}$ | + |
| C13 short | NS1$_{(1-42)}$-M-D-L-S-R-HA2$_{(1-222)}$ | + |
| D short | NS1$_{(1-42)}$-M-D-H-M-L-T-S-T-R-S-HA2$_{(66-222)}$ | + |
| A | NS1$_{(1-81)}$-Q-I-P-HA2$_{(69-222)}$ | + |
| C | NS1$_{(1-81)}$-Q-I-P-HA2$_{(81-222)}$ | + |
| ΔD | NS1$_{(1-81)}$-Q-I-P-V-HA2$_{(150-222)}$ | + |
| Δ13 | NS1$_{(1-81)}$-D-L-S-R-HA2$_{(1-70)}$-S-C-L-T-A-Y-H-R | - |
| M | NS1$_{(1-81)}$-Q-I-P-HA2$_{(65-196)}$-G-G-S-Y-S-M-E-H-F-R-W-G-K-P-V | - |
| ΔM | NS1$_{(1-81)}$-Q-I-P-HA2$_{(65-196)}$-G-G-S-Y-S-M-L-V-N | - |

Example 20. In Vivo Induction of Antiviral CTL

In the following example, D protein, delta M protein and delta M+ protein (described above in Examples 4, 12, and 13, respectively) were tested for their ability to induce antiviral cytotoxic T-lymphocytes in vivo.

Mice ((Balb/c x C57BL/6)F₁) were immunized with D protein, delta M protein or delta M+ protein in complete Freund's adjuvant (CFA). D protein was purified according to Example 15 (purity around 90%). Delta M protein and delta M+ protein were each purified according to the method described for the purification of C13 protein in Example 3, above (purity around 50%).

For each antigen tested, mice were divided into three groups (3 mice per group), the first group receiving 10 ug total protein, the second group receiving 50 ug total protein, and a control group receiving CFA only. Each mouse received two injections, one at the base of the tail (0.1 ml) and one in the hind foot pad (0.1 ml). One week after the injections, lymph nodes surrounding the injection sites were removed from each mouse. Nodes from all mice in a group were pooled. Single cell suspensions were prepared by passing the lymph nodes through a stainless steel mesh. The resulting cells were washed twice and resuspended in complete medium (RPM1 1640 + % Fetal Calf Serum, 2 mM glutamine, 10 mM Hepes buffer 5 x $10^{-5}$ M 2-mercaptoethanol, penicillin and streptomycin).

Immune lymph node cells (6 x $10^6$ cells) were stimulated with $10^6$ A/PR/8 infected normal spleen cells (as described below) for 5 days at 37°C in 6% $CO_2$. Cultures were set up in 24-well plates, 2 ml total volume per well. The cultured cells ("CTL effector cells") were then harvested, washed twice, and resuspended in complete medium at appropriate concentrations for the CTL (chromium release) assay described below.

To prepare virus infected stimulator cells, spleens were asceptically removed from (Balb/c x C57BL/6)-F₁ mice and teased through a stainless steel mesh to prepare single cell suspensions. Red blood cells were lysed by hypotonic shock. Cells were washed twice and resuspended in complete medium at 6 x $10^6$ cells per ml. Cells were infected with A/PR/8/34 virus (around 20 plaque forming units (PFU) per cell) for one hour at 37° C in 5% $CO_2$ with gentle shaking at 15 minute intervals. (A/PR/8/34 (H1N1) and B/Lee/40 influenza viruses were grown in 9 day old embryonated chicken eggs for 48 hours. Allantoic fluid from infected eggs was harvested, pooled, and stored in aliquots at -70C). Cells were then washed twice and adjusted to 1 X $10^6$ cells/ml in complete medium. 1 ml of the infected cell suspension was added to wells containing 6 X $10^6$ immune lymph node cells.

The CTL assays were performed as follows. P815 cells (a mastocytoma line derived from DBA mice, maintained in suspension culture in Eagle's Minimum Essential Medium (MEM) supplemented with 10% fetal calf serum and 2 mM glutamine) in log phase of growth were used as target cells in the chromium release assay. P815 cells were labelled with $Na_2CRO_4$ ($Cr^{51}$) (300 uCi per $10^7$ cells) in the absence of serum for 30 minutes at 37° C, 6% $CO_2$. Virus (around 10 PFU per cell) was then added and the incubation continued for an additional hour. Cells were then washed twice, resuspended in complete medium (1-2 X $10^6$ cells per ml) and reincubated for 3.5 hours at 37° C. After washing the cells twice, they were adjusted to 1 X $10^5$ cells per ml and 0.1 ml thereof added to round bottomed microwells (96 well plate) containing 0.1 ml CTL effector cells so that the final effector to target cell ratios were 50:1, 25:1; 12.5:1; and 6.25:1 (all set up in triplicate wells). The plates were centrifuged for 5 minutes at 600 rpm and then incubated for four hours at 37° C in 5% $CO_2$. The amount of $^{51}$Cr released was measured by sampling 0.1 ml of supernatant form each culture for counting in a gamma counter (Beckman Instruments Gamma 8000).

Percent cytotoxicity was calculated by the formula (E-C/T-C) X 100 where E = counts per minute released in the presence of effector cells, C = counts per minute released by target cells incubated with 0.1 ml complete medium (spontaneous release) and T = total counts per minute per well. For total counts per minute, 0.1 ml target cells were incubated for 3.5 hours with 0.1 ml 1% sodium dodecyl sulfate and the contents of the well mixed before sampling 0.1 ml.

In conducting the proliferation assay, cells from immune lymph nodes (0.2 ml) were added to flat bottomed wells of a 96 well microtiter plate at 4 X $10^5$ cells per well. Cells were stimulated with 10 ug per ml of D protein (90% purity) for 72 hours at 37° C in 6% $CO_2$. (Stock preparations of the D protein were appropriately diluted with 5% dextrose in water before adding 25 ul per well.) The wells were then pulsed with $^3$H thymidine (1 uCi) for the final six hours of culture and then collected onto filters with an automatic cell harvester (Scatron) for scintillation counting.

Results are presented in Table 11, below.

Table 11

| In vivo CTL Induction | | | | | | | |
| --- | --- | --- | --- | --- | --- | --- | --- |
| | | % Specific Lysis* | | | | | |
| | | A/PR8 | | | B/Lee | | |
| ( In Vivo Protein) | Dose | 12:1 | 25:1 | 50:1 | 12:1 | 25:1 | 50:1 |
| D | 10 | 29 | 35 | 36 | 7 | 8 | 8 |
| | 50 | 22 | 30 | 38 | 4 | 5 | 8 |
| ΔM | 10 | 7 | 19 | 24 | 6 | 10 | 13 |
| | 50 | 19 | 25 | 33 | 3 | 11 | ˙19 |
| ΔM+ | 10 | 25 | 38 | 51 | 1 | <1 | 4 |
| | 50 | 48 | 52 | 54 | 6 | 5 | 3 |
| CFA | | 1 | 12 | 16 | 7 | 6 | 5 |

*Represents percent cytotoxicity against virus-infected target minus percent cytotoxicity against uninfected target. Responses are positive when % specific lysis is greater than or equal to two-fold that seen in cells from mice imm nized with CFA only.

The data show that delta M+ protein effectively induced a Type A specific cytotoxic T-lymphocyte response at both doses (10 ug and 50 ug) tested. Delta M protein induced a positive CTL response at the higher dose (50 ug) although the CTL activity was not solely directed against the Type A infected targets as type B infected targets were also killed. Because CTLs directed against similar recombinant influenza proteins (Kuwano et al., 1988. J. Immunol. 140:1264-1268) or against virus (Yap et al., 1978. Nature 273:238-239) can adoptively transfer protective immunity in the mouse model and, further, are believed to play a role in viral clearance of human influenza virus (McMichael et al., 1983. N.Engl. J. Med. 309:13-17), either of these two proteins are candidates for a human influenza vaccine.

Cells from mice immunized with either D protein, delta M or delta M+ were also tested for proliferative response to 10 ug/ml D protein in vitro. The response of all groups was strongly positive (P <0.001 by the two-tailed Student's T test vs. the response in the CFA control group). In vitro proliferation in response to influenza derived proteins is an established property of T helper cell clones which can augment or support influenza-specific antibody production by B cells (Scherle and Gerhard, 1986. J.Exp. Med. 164:1114-1128). Since production of neutralizing antibodies can be facilitated by helper T cells in vivo (Scherle and Gerhard, 1986, supra, and 1988, PNAS 4446 4450; Tite et al., 1988 J. Immunol 141:3980-3987) proteins which induce cells with potential T helper cell activity are also considered candidates for human vaccine immunogens.

The invention and its preferred embodiments are fully disclosed above. However, the invention is not limited to such specifically disclosed embodiments. Rather, it encompasses all modifications and variations coming within the scope of the following claims.

## Claims

1. A process for purifying an immunogenic polypeptide from the cell lysate of a recombinant host cell culture, said polypeptide comprising an immunogenic determinant of the HA2 subunit of an influenza virus HA protein and being insoluble in the cell lysate, the process comprising:

subjecting the cell lysate to a first detergent treatment, at a pH in the range between about 6 to about 8.5, to selectively solubilize host cell contaminants and forming thereby a soluble fraction and an insoluble fraction, the insoluble fraction containing the polypeptide;

separating the soluble fraction from the insoluble fraction;

subjecting the insoluble fraction to a second detergent treatment, at a pH in the range between about 9.5 to about 11, to selectively solubilize host cell contaminants and forming thereby a soluble fraction and aan insoluble fraction, the insoluble fraction containing the polypeptide;

separating the soluble fraction from the insoluble fraction;

subjecting the insoluble fraction to a chaotropic agent thereby solubilizing the polypeptide;

separating the soluble fraction from the insoluble fraction, the soluble fraction containing the polypeptide;

adding a reducing agent to the soluble fraction;

subjecting the soluble fraction to ion exchange chromatography and recovering a polypeptide containing eluate, said eluate being substantially free of contaminating host cell nucleic acids and polypeptides.

2. The process of Claim 1 wherein the detergent of the first detergent treatment comprises an ionic detergent.

3. The process of Claim 2 wherein the ionic detergent is deoxycholate.

4. The process of Claim 1 wherein the first detergent treatment is performed at a pH of about 8.

5. The process of Claim 1 wherein the detergent of the second detergent treatment comprises a non-ionic detergent.

6. The process of Claim 1 wherein the second detergent treatment is performed at pH of about 10.5.

7. The process of Claim 1 wherein the chaotropic agent is urea at a pH in the range of between about 7.5 to about 9.

8. The process of Claim 1 wherein the chromatography is anion exchange chromatography.

9. The process of Claim 1 which further comprises diafiltration of the polypeptide-containing eluate.

10. The process of Claim 1 which further comprises treating the immunogenic polypeptide-containing fractions recovered from ion exchange chromatography to a strong denaturant under reducing conditions, subjecting the thus-treated fractions to size exclusion chromatography and recovering the resulting immunogenic polypeptide-containing eluate, substantially free of host cell endotoxins.

11. The process of Claim 10, further comprising a second size exclusion chromatography step, whereby the immunogenic polypeptide-containing fraction recovered from the size exclusion chromatography is applied to a second size exclusion chromatograph in the presence of a chaotropic agent and the resulting immunogenic polypeptide-containing fractions are recoverd substantially free of denaturant.

12. The process of Claim 10 wherein the denaturant is sodium dodecylsulfate.

13. The process of Claim 11 wherein the chaotropic agent is urea.

14. The process of Claim 11 wherein the second size exclusion chromatography step is performed on a desalting chromatograph.

→NSI
```
ATGGATCCAAACACTGTGTCAAGCTTTCAGGTAGATTGCTTTCTTTGGCATGTCCGCAAA
+---------+---------+---------+---------+---------+---------
TACCTAGGTTTGTGACACAGTTCGAAAGTCCATCTAACGAAAGAAACCGTACAGGCGTTT
```
MetAspProAsnThrValSerSerPheGlnValAspCysPheLeuTrpHisValArgLys

```
CGAGTTGCAGACCAAGAACTAGGTGATGCCCCATTCCTTGATCGGCTTCGCCGAGATCAG
+---------+---------+---------+---------+---------+---------
GCTCAACGTCTGGTTCTTGATCCACTACGGGGTAAGGAACTAGCCGAAGCGGCTCTAGTC
```
ArgValAlaAspGlnGluLeuGlyAspAlaProPheLeuAspArgLeuArgArgAspGln

```
AAATCCCTAAGAGGAAGGGGCAGCACTCTTGGTCTGGACATCGAGACAGCCACACGTGCT
+---------+---------+---------+--·--·----+---------+---------
TTTAGGGATTCTCCTTCCCCGTCGTGAGAACCAGACCTGTAGCTCTGTCGGTGTGCACGA
```
LysSerLeuArgGlyArgGlySerThrLeuGlyLeuAspIleGluThrAlaThrArgAla

```
GGAAAGCAGATAGTGGAGCGGATTCTGAAAGAAGAATCCGATGAGGCACTTAAAATGACC
+---------+---------+---------+---------+---------+---------
CCTTTCGTCTATCACCTCGCCTAAGACTTTCTTCTTAGGGTACTCCGTGAATTTTACTGG
```
GlyLysGlnIleValGluArgIleLeuLysGluGluSerAspGluAlaLeuLysMetThr

→IN2
```
ATGGATCTGTCCAGAGGTCTATTTGGAGCCATTGCCGGTTTTATTGAAGGGGGATGGACT
+--------+----+---------+---------+---------+---------+-----
TACCTAGACAGGTCTCCAGATAAACCTCGGTAACGGCCAAAATAACTTCCCCCTACCTGA
```
BI  linker 316 327  /
MetAspLeuSerArgGlyLeuPheGlyAlaIleAlaGlyPheIleGluGlyGlyTrpThr

```
GGAATGATAGATGGATGGTACGGTTATCATCATCAGAATGAACAGGGATCAGGCTATGCA
+---------+---------+---------+---------+---------+---------
CCTTACTATCTACCTACCATGCCAATAGTAGTAGTCTTACTTGTCCCTAGTCCGATACGT
```
GlyMetIleAspGlyTrpTyrGlyTyrHisHisGlnAsnGluGlnGlySerGlyTyrAla

```
GCGGATCAAAAAAGCACACAAAATGCCATTAACGGGATTACAAACAAGGTGAACTCTGTT
+---------+---------+---------+---------+---------+---------
CGCCTAGTTTTTTCGTGTGTTTTACGGTAATTGCCCTAATGTTTGTTCCACTTGAGACAA
```
AlaAspGlnLysSerThrGlnAsnAlaIleAsnGlyIleThrAsnLysValAsnSerVal

```
ATCGAGAAAATGAACATTCAATTCACAGCTGTGGGTAAAGAATTCAACAAATTAGAAAAA
+---------+---------+---------+---------+---------+---------
TAGCTCTTTTACTTGTAAGTTAAGTGTCGACACCCATTTCTTAAGTTGTTTAATCTTTTT
```
IleGluLysMetAsnIleGlnPheThrAlaValGlyLysGluPheAsnLysLeuGluLys

```
AGGATGGAAAATTTAAATAAAAAAGTTGATGATGGATTCTGGACATTTGGACATATAAT
+---------+---------+---------+---------+---------+---------
TCCTACCTTTTAAATTTATTTTTTCAACTACTACCTAAAGACCTGTAAACCTGTATATTA
```
ArgMetGluAsnLeuAsnLysLysValAspAspGlyPheLeuAspIleTrpThrTyrAsn

```
GCAGAATTGTTAGTTCTACTGGAAAATGAAAGGACTCTGGATTTCCATGACTCAAATGTG
+---------+---------+---------+---------+---------+---------
CGTCTTAACAATCAAGATGACCTTTTACTTTCCTGAGACCTAAAGGTACTGAGTTTACAC
```
AlaGluLeuLeuValLeuLeuGluAsnGluArgThrLeuAspPheHisAspSerAsnVal

```
AAGAATCTGTATGAGAAAGTAAAAAGCCAATTAAAGAATAATGCCAAAGAAATCGGAAAT
+---------+---------+---------+---------+---------+---------
TTCTTAGACATACTCTTTCATTTTTCGGTTAATTTCTTATTACGGTTTCTTTAGCCTTTA
```
LysAsnLeuTyrGluLysValLysSerGlnLeuLysAsnAsnAlaLysGluIleGlyAsn

```
GGATGTTTTGAGTTCTACCACAAGTGTGACAATGAATGCATGGAAAGTGTAAGAAATGGG
+---------+---------+---------+---------+---------+---------
CCTACAAAACTCAAGATGGTGTTCACACTGTTACTTACGTACCTTTCACATTCTTTACCC
```
GlyCysPheGluPheTyrHisLysCysAspAsnGluCysMetGluSerValArgAsnGly

```
ACTTATGATTATCCCAAATATTCAGAAGAGTCAAAGTTGAACAGGGAAAAGGTAGATGGA
+---------+---------+---------+---------+---------+---------
TGAATACTAATAGGGTTTATAAGTCTTCTCAGTTTCAACTTGTCCCTTTTCCATCTACCT
```
ThrTyrAspTyrProLysTyrSerGluGluSerLysLeuAsnArgGluLysValAspGly

```
GTGAAATTGGAATCAATGGGGATCTATCAGATTCTGGCGATCTACTCAACTGTCGCCAGT
+---------+---------+---------+----·-----+---------+---------
CACTTTAACCTTAGTTACCCCTAGATAGTCTAAGACCGCTAGATGAGTTGACAGCGGTCA
```
ValLysLeuGluSerMetGlyIleTyrGlnIleLeuAlaIleTyrSerThrValAlaSer

```
TCACTGGTGCTTTTGGTCTCCCTGGGGGCAATCAGTTTCTGGATGTGTTCTAATGGATCT
+---------+---------+---------+---------+---------+---------
AGTGACCACGAAAACCAGAGGGACCCCCGTTAGTCAAAGACCTACACAAGATTACCTAGA
```
SerLeuValLeuLeuValSerLeuGlyAlaIleSerPheTrpMetCysSerAsnGlySer

```
TTGCAGTGCAGAATATGCATCTGA
+---------+---------+---
AACGTCACGTCTTATACGTAGACT
```
LeuGlnCysArgIleCysIleEnd -

**FIGURE 1**

→NSI

```
ATGGATCCAAACACTGTGTCAAGCTTTCAGGTAGATTGCTTTCTTTGGCATGTCCGCAAA
------+---------+---------+---------+---------+---------+---
TACCTAGGTTTGTGACACAGTTCGAAAGTCCATCTAACGAAAGAAACCGTACAGGCGTTT
```
MetAspProAsnThrValSerSerPheGlnValAspCysPheLeuTrpHisValArgLys

```
CGAGTTGCAGACCAAGAACTAGGTGATGCCCCATTCCTTGATCGGCTTCGCCGAGATCAG
------+---------+---------+---------+---------+---------+---
GCTCAACGTCTGGTTCTTGATCCACTACGGGGTAAGGAACTAGCCGAAGCGGCTCTAGTC
```
ArgValAlaAspGlnGluLeuGlyAspAlaProPheLeuAspArgLeuArgArgAspGln

```
AAATCCCTAAGAGGAAGGGGGCAGCACTCTTGGTCTGGACATCGAGACAGCCACACGTGCT
------+---------+---------+---------+---------+---------+---
TTTAGGGATTCTCCTTCCCCGTCGTGAGAACCAGACCTGTAGCTCTGTCGGTGTGCACGA
```
LysSerLeuArgGlyArgGlySerThrLeuGlyLeuAspIleGluThrAlaThrArgAla

```
GGAAAGCAGATAGTGGAGCGGATTCTGAAAGAAGAATCCGATGAGGCACTTAAAATGACC
------+---------+---------+---------+---------+---------+---
CCTTTCGTCTATCACCTCGCCTAAGACTTTCTTCTTAGGCTACTCCGTGAATTTTACTGG
```
GlyLysGlnIleValGluArgIleLeuLysGluGluSerAspGluAlaLeuLysMetThr

→HA2
```
ATGCAGATCCCGGCTGTGGGTAAAGAATTCAACAAATTAGAAAAAAGGATGGAAAATTTA
------+---------+---------+---------+---------+---------+---
TACGTCTAGGGCCGACACCCATTTCTTAAGTTGTTTAATCTTTTTTCCTACCTTTTAAAT
```
81│ linker │65          69
MetGlnIleProAlaValGlyLysGluPheAsnLysLeuGluLysArgMetGluAsnLeu

```
AATAAAAAAGTTGATGATGGATTTCTGGACATTTGGACATATAATGCAGAATTGTTAGTT
------+---------+---------+---------+---------+---------+---
TTATTTTTTCAACTACTACCTAAAGACCTGTAAACCTGTATATTACGTCTTAACAATCAA
```
81
AsnLysLysValAspAspGlyPheLeuAspIleTrpThrTyrAsnAlaGluLeuLeuVal

```
CTACTGGAAAATGAAAGGACTCTGGATTTCCATGACTCAAATGTGAAGAATCTGTATGAG
------+---------+---------+---------+---------+---------+---
GATGACCTTTTACTTTCCTGAGACCTAAAGGTACTGAGTTTACACTTCTTAGACATACTC
```
LeuLeuGluAsnGluArgThrLeuAspPheHisAspSerAsnValLysAsnLeuTyrGlu

```
AAAGTAAAAAGCCAATTAAAGAATAATGCCAAAGAAATCGGAAATGGATGTTTTGAGTTC
------+---------+---------+---------+---------+---------+---
TTTCATTTTTCGGTTAATTTCTTATTACGGTTTCTTTAGCCTTTACCTACAAAACTCAAG
```
LysValLysSerGlnLeuLysAsnAsnAlaLysGluIleGlyAsnGlyCysPheGluPhe

```
TACCACAAGTGTGACAATGAATGCATGGAAAGTGTAAGAAATGGGACTTATGATTATCCC
------+---------+---------+---------+---------+---------+---
ATGGTGTTCACACTGTTACTTACGTACCTTTCACATTCTTTACCCTGAATACTAATAGGG
```
150
TyrHisLysCysAspAsnGluCysMetGluSerValArgAsnGlyThrTyrAspTyrPro

```
AAATATTCAGAAGAGTCAAAGTTGAACAGGGAAAAGGTAGATGGAGTGAAATTGGAATCA
------+---------+---------+---------+---------+---------+---
TTTATAAGTCTTCTCAGTTTCAACTTGTCCCTTTTCCATCTACCTCACTTTAACCTTAGT
```
LysTyrSerGluGluSerLysLeuAsnArgGluLysValAspGlyValLysLeuGluSer

```
ATGGGGATCTATCAGATTCTGGCGATCTACTCAACTGTCGCCAGTTCACTGGTGCTTTTG
------+---------+---------+---------+---------+---------+---
TACCCCTAGATAGTCTAAGACCGCTAGATGAGTTGACAGCGGTCAAGTGACCACGAAAAC
```
MetGlyIleTyrGlnIleLeuAlaIleTyrSerThrValAlaSerSerLeuValLeuLeu

```
GTCTCCCTGGGGGCAATCAGTTTCTGGATGTGTTCTAATGGATCTTTGCAGTGCAGAATA
------+---------+---------+---------+---------+---------+---
CAGAGGGACCCCCGTTAGTCAAAGACCTACACAAGATTACCTAGAAACGTCACGTCTTAT
```
ValSerLeuGlyAlaIleSerPheTrpMetCysSerAsnGlySerLeuGlnCysArgIle

```
TGCATCTGA
------+--
ACGTAGACT
```
222
CysIleEnd -

## FIGURE 2

EP 0 366 239 A1

→ NSI
```
ATGGATCCAAACACTGTGTCAAGCTTTCAGGTAGATTCCTTTCTTTGGCATGTCCGCAAA
•---------+---------+---------+---------+---------+---------
TACCTAGGTTTGTGACACAGTTCGAAAGTCCATCTAAGGAAAGAAACCGTACAGGCGTTT
```
/
MetAspProAsnThrValSerSerPheGlnValAspSerPheLeuTrpHisValArgLys

```
CGAGTTGCAGACCAAGAACTAGGTGATGCCCCATTCCTTGATCGGCTTCGCCGAGATCAG
•---------+---------+---------+---------+---------+---------
GCTCAACGTCTGGTTCTTGATCCACTACGGGGTAAGGAACTAGCCGAAGCGGCTCTAGTC
```
ArgValAlaAspGlnGluLeuGlyAspAlaProPheLeuAspArgLeuArgArgAspGln

→ HA2
```
AAATCCATGGATCTGTCCAGAGGTCTATTTGGAGCCATTGCCGGTTTTATTGAAGGGGGA
•----+---+-----+---------+---------+---------+---------+-----
TTTAGGTACCTAGACAGGTCTCCAGATAAACCTCGGTAACGGCCAAAATAACTTCCCCCT
```
42    linker    326 327   /
LysSerMetAspLeuSerArgGlyLeuPheGlyAlaIleAlaGlyPheIleGluGlyGly

```
TGGACTGGAATGATAGATGGATGGTACGGTTATCATCATCAGAATGAACAGGGATCAGGC
•---------+---------+---------+---------+---------+---------
ACCTGACCTTACTATCTACCTACCATGGCAATAGTAGTAGTCTTACTTGTCCCTAGTCCG
```
TrpThrGlyMetIleAspGlyTrpTyrGlyTyrHisHisGlnAsnGluGlnGlySerGly

```
TATGCAGCGGATCAAAAAAGCACACAAAATGCCATTAACGGGATTACAAACAAGGTGAAC
•---------+---------+---------+---------+---------+---------
ATACGTCGCCTAGTTTTTTCGTGTGTTTTACGGTAATTGCCCTAATGTTTGTTCCACTTG
```
TyrAlaAlaAspGlnLysSerThrGlnAsnAlaIleAsnGlyIleThrAsnLysValAsn

```
TCTGTTATCGAGAAAATGAACATTCAATTCACAGCTGTGGGTAAAGAATTCAACAAATTA
•---------+---------+---------+---------+---------+---------
AGACAATAGCTCTTTTACTTGTAAGTTAAGTGTCGACACCCATTTCTTAAGTTGTTTAAT
```
SerValIleGluLysMetAsnIleGlnPheThrAlaValGlyLysGluPheAsnLysLeu

```
GAAAAAAGGATGGAAAATTTAAATAAAAAAGTTGATGATGGATTTCTGGACATTTGGACA
•---------+---------+---------+---------+---------+---------
CTTTTTTCCTACCTTTTAAATTTATTTTTTCAACTACTACCTAAAGACCTGTAAACCTGT
```
GluLysArgMetGluAsnLeuAsnLysLysValAspAspGlyPheLeuAspIleTrpThr

```
TATAATGCAGAATTGTTAGTTCTACTGGAAAATGAAAGGACTCTGGATTTCCATGACTCA
•---------+---------+---------+---------+---------+---------
ATATTACGTCTTAACAATCAAGATGACCTTTTACTTTCCTGAGACCTAAAGGTACTGAGT
```
TyrAsnAlaGluLeuLeuValLeuLeuGluAsnGluArgThrLeuAspPheHisAspSer

```
AATGTGAAGAATCTGTATGAGAAAGTAAAAAAGCCAATTAAAGAATAATGCCAAAGAAATC
•---------+---------+---------+---------+---------+---------
TTACACTTCTTAGACATACTCTTTCATTTTTCGGTTAATTTCTTATTACGGTTTCTTTAG
```
AsnValLysAsnLeuTyrGluLysValLysSerGlnLeuLysAsnAsnAlaLysGluIle

```
GGAAATGGATGTTTTGAGTTCTACCACAAGTGTGACAATGAATGCATGGAAAGTGTAAGA
•---------+---------+---------+---------+---------+---------
CCTTTACCTACAAAACTCAAGATGGTGTTCACACTGTTACTTACGTACCTTTCACATTCT
```
GlyAsnGlyCysPheGluPheTyrHisLysCysAspAsnGluCysMetGluSerValArg

```
AATGGGACTTATGATTATCCCAAATATTCAGAAGAGTCAAAGTTGAACAGGGAAAAGGTA
•---------+---------+---------+---------+---------+---------
TTACCCTGAATACTAATAGGGTTTATAAGTCTTCTCAGTTTCAACTTGTCCCTTTTCCAT
```
AsnGlyThrTyrAspTyrProLysTyrSerGluGluSerLysLeuAsnArgGluLysVal

```
GATGGAGTGAAATTGGAATCAATGGGGATCTATCAGATTCTGGCGATCTACTCAACTGTC
•---------+---------+---------+---------+---------+------•--
CTACCTCACTTTAACCTTAGTTACCCCTAGATAGTCTAAGACCGCTAGATGAGTTGACAG
```
AspGlyValLysLeuGluSerMetGlyIleTyrGlnIleLeuAlaIleTyrSerThrVal

```
GCCAGTTCACTGGTGCTTTTGGTCTCCCTGGGGGGCAATCAGTTTCTGGATGTGTTCTAAT
•---------+---------+---------+---------+---------•---------
CGGTCAAGTGACCACGAAAACCAGAGGGACCCCCGTTAGTCAAAGACCTACACAAGATTA
```
AlaSerSerLeuValLeuLeuValSerLeuGlyAlaIleSerPheTrpMetCysSerAsn

```
GGATCTTTGCAGTGCAGAATATGCATCTGA
•---------+---------+--------•
CCTAGAAACGTCACGTCTTATACGTAGACT
```
                    121
GlySerLeuGlnCysArgIleCysIleEnd -

FIGURE 3

➤NSI
```
ATGGATCCAAACACTGTGTCAAGCTTTCAGGTAGATTCCTTTCTTTGGCATGTCCGCAAA
-------+---------+---------+---------+---------+---------+--
TACCTAGGTTTGTGACACAGTTCGAAAGTCCATCTAAGGAAAGAAACCGTACAGGCGTTT
```
│
MetAspProAsnThrValSerSerPheGlnValAspSerPheLeuTrpHisValArgLys

```
CGAGTTGCAGACCAAGAACTAGGTGATGCCCCATTCCTTGATCGGCTTCGCCGAGATCAG
-------+---------+---------+---------+---------+---------+--
GCTCAACGTCTGGTTCTTGATCCACTACGGGGTAAGGAACTAGCCGAAGCGGCTCTAGTC
```

ArgValAlaAspGlnGluLeuGlyAspAlaProPheLeuAspArgLeuArgArgAspGln

➤HA2
```
AAATCCATGGATCATATGTTAACAAGTACTCGATCTGTGGGTAAAGAATTCAACAAATTA
-------+---------+---------+---------+---------+---------+--
TTTAGGTACCTAGTATACAATTGTTCATGAGCTAGACACCCATTTCTTAAGTTGTTTAAT
```
1/1              linker                66
LysSerMetAspHisMetLeuThrSerThrArgSerValGlyLysGluPheAsnLysLeu

```
GAAAAAAGGATGGAAAATTTAAATAAAAAAGTTGATGATGGATTTCTGGACATTTGGACA
-------+---------+---------+---------+---------+---------+--
CTTTTTTCCTACCTTTTAAATTTATTTTTTCAACTACTACCTAAAGACCTGTAAACCTGT
```

GluLysArgMetGluAsnLeuAsnLysLysValAspAspGlyPheLeuAspIleTrpThr

```
TATAATGCAGAATTGTTAGTTCTACTGGAAAATGAAAGGACTCTGGATTTCCATGACTCA
-------+---------+---------+---------+---------+---------+--
ATATTACGTCTTAACAATCAAGATGACCTTTTACTTTCCTGAGACCTAAAGGTACTGAGT
```

TyrAsnAlaGluLeuLeuValLeuLeuGluAsnGluArgThrLeuAspPheHisAspSer

```
AATGTGAAGAATCTGTATGAGAAAGTAAAAAGCCAATTAAAGAATAATGCCAAAGAAATC
-------+---------+---------+---------+---------+---------+--
TTACACTTCTTAGACATACTCTTTCATTTTTCGGTTAATTTCTTATTACGGTTTCTTTAG
```

AsnValLysAsnLeuTyrGluLysValLysSerGlnLeuLysAsnAsnAlaLysGluIle

```
GGAAATGGATGTTTTGAGTTCTACCACAAGTGTGACAATGAATGCATGGAAAGTGTAAGA
-------+---------+---------+---------+---------+---------+--
CCTTTACCTACAAAACTCAAGATGGTGTTCACACTGTTACTTACGTACCTTTCACATTCT
```

GlyAsnGlyCysPheGluPheTyrHisLysCysAspAsnGluCysMetGluSerValArg

```
AATGGGACTTATGATTATCCCAAATATTCAGAAGAGTCAAAGTTGAACAGGGAAAAGGTA
-------+---------+---------+---------+---------+---------+--
TTACCCTGAATACTAATAGGGTTTATAAGTCTTCTCAGTTTCAACTTGTCCCTTTTCCAT
```

AsnGlyThrTyrAspTyrProLysTyrSerGluGluSerLysLeuAsnArgGluLysVal

```
GATGGAGTGAAAATTGGAATCAATGGGGATCTATCAGATTCTGGCGATCTACTCAACTGTC
-------+---------+---------+---------+---------+---------+--
CTACCTCACTTTAACCTTAGTTACCCCTAGATAGTCTAAGACCGCTAGATGAGTTGACAG
```

AspGlyValLysLeuGluSerMetGlyIleTyrGlnIleLeuAlaIleTyrSerThrVal

```
GCCAGTTCACTGGTGCTTTTGGTCTCCCTGGGGGGCAATCAGTTTCTGGATGTGTTCTAAT
-------+---------+---------+---------+---------+---------+--
CGGTCAAGTGACCACGAAAACCAGAGGGACCCCCGTTAGTCAAAGACCTACACAAGATTA
```

AlaSerSerLeuValLeuLeuValSerLeuGlyAlaIleSerPheTrpMetCysSerAsn

```
GGATCTTTGCAGTGCAGAATATGCATCTGA
-------+---------+---------+--
CCTAGAAACGTCACGTCTTATACGTAGACT
```
222
GlySerLeuGlnCysArgIleCysIleEnd

# FIGURE 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 176 493 (SMITHKLINE BECKMAN) * Page 10, line 7 - page 11, line 11 * | 1-14 | C 12 P 21/02 A 61 K 39/145// C 12 N 15/44 |
| Y | FR-A-2 477 417 (INSTITUT PASTEUR) * Page 5, lines 7-27; page 10, line 18 - page 11, line 30 * | 1-14 | |
| D,A | PROC. NATL. ACAD. SCI. USA, vol. 80, October 1983, pages 6105-6109; J.F. YOUNG et al.: "Efficient expression of influenza virus NS1 nonstructural proteins in Escherichia coli" | | |

TECHNICAL FIELDS SEARCHED (Int. Cl.5)

C 12 P
A 61 K

The present search report has been drawn up for all claims

| Place of search | Date of completion of the search | Examiner |
|---|---|---|
| THE HAGUE | 06-12-1989 | SKELLY J.M. |

EPO FORM 1503 03.82 (P0401)